# EUROPEAN PATENT SPECIFICATION

(11) **EP 3 923 963 B1**
(45) Date of publication and mention of the grant of the patent: **10.06.2026**
(21) Application number: 20756733.0
(22) Date of filing: 14.02.2020
(51) Int. Cl.: A61K 35/34, A61K 35/12, A61K 9/00, A61P 9/04

(54) **TREATING HEART FAILURE**
BEHANDLUNG VON HERZVERSAGEN
TRAITEMENT D'UNE INSUFFISANCE CARDIAQUE

(30) Priority: 15.02.2019 US 201962806473 P
(43) Date of publication of application: 22.12.2021
(73) Proprietor: CHILDREN'S MEDICAL CENTER CORPORATION, Boston, Massachusetts 02115 (US)
(72) Inventor: MCCULLY, James D., Marblehead, Massachusetts 01945 (US)
(74) Representative: Fish & Richardson P.C.
(86) International application number: PCT/US2020/018371
(87) International publication number: WO 2020/168247

(56) References cited:
- WO-A1-2017/095940
- WO-A1-2017/124037
- CA-A1- 3 049 099
- US-A1- 2007 015 777
- US-A1- 2009 202 606
- US-A1- 2018 340 022
- WEIXLER: "Thieme E-Journals - The Thoracic and Cardiovascular Surgeon / Full Text", 17 February 2019 (2019-02-17) - 17 February 2019 (2019-02-17), XP055970479, Retrieved from the Internet <URL:https://www.thieme-connect.de/products/ejournals/html/10.1055/s-0039-1678837> [retrieved on 20221012]
- DOUGLAS B. COWAN ET AL: "Intracoronary Delivery of Mitochondria to the Ischemic Heart for Cardioprotection", PLOS ONE, vol. 11, no. 8, 8 August 2016 (2016-08-08), pages e0160889, XP055720563, DOI: 10.1371/journal.pone.0160889
- ROUSHANDEH AMANEH MOHAMMADI ET AL: "Mitochondrial transplantation as a potential and novel master key for treatment of various incurable diseases", CYTOTECHNOLOGY, SPRINGER NETHERLANDS, DORDRECHT, vol. 71, no. 2, 31 January 2019 (2019-01-31), pages 647 - 663, XP036759622, ISSN: 0920-9069, [retrieved on 20190131], DOI: 10.1007/S10616-019-00302-9
- BAYEVA MARINA ET AL: "Mitochondria as a Therapeutic Target in Heart Failure", JOURNAL OF THE AMERICAN COLLEGE OF CARDIOLOGY, ELSEVIER, AMSTERDAM, NL, vol. 61, no. 6, 5 December 2012 (2012-12-05), pages 599 - 610, XP028969906, ISSN: 0735-1097, DOI: 10.1016/J.JACC.2012.08.1021
- MCMURRAY J J ET AL: "Heart failure", THE LANCET, ELSEVIER, AMSTERDAM, NL, vol. 365, no. 9474, 28 May 2005 (2005-05-28), pages 1877 - 1889, XP025278097, ISSN: 0140-6736, [retrieved on 20050528], DOI: 10.1016/S0140-6736(05)66621-4
- LIN PIAO ET AL: "Mitochondrial metabolic adaptation in right ventricular hypertrophy and failure", JOURNAL OF MOLECULAR MEDICINE, SPRINGER, BERLIN, DE, vol. 88, no. 10, 4 September 2010 (2010-09-04), pages 1011 - 1020, XP019823122, ISSN: 1432-1440
- SHIN ET AL.: "A Novel Biological Strategy for Myocardial Protection by intracoronary Delivery of Mitochondria: Safety and Efficacy", JACC: BASIC TO TRANSLATIONAL SCIENCE, vol. 4, no. 8, December 2019 (2019-12-01), pages 871 - 888, XP055733280, Retrieved from the Internet <URL:http://basictranslational.onlinejacc.org/content4/8/871> [retrieved on 20200414]

## Description

### FIELD

The disclosure relates to mitochondria for therapeutic use and combined mitochondrial agents for therapeutic use.

### BACKGROUND

Mitochondria are double membrane-bound organelles found in the cytoplasm of nucleated eukaryotic cells. They are found in almost every cell of the human body except red blood cells. They are the cell's primary site of energy metabolism and generate adenosine triphosphate (ATP) for different cell functions. Typically, more than 90% of a cell's requirement for ATP is supplied by the cell's own mitochondria.

Mitochondria are composed of two concentric membranes, which have specialized functions. The inner mitochondrial membrane contains proteins for ATP synthase. The outer mitochondrial membrane, which contains large numbers of integral membrane proteins, encloses the entire organelle.

The structure of mitochondria has striking similarities to some modem prokaryotes. In fact, mitochondria are thought to have originated from an ancient symbiosis when a nucleated cell engulfed an aerobic prokaryote. In the symbiosis relationship, the host cell came to rely on the engulfed prokaryote for energy production, and the prokaryote cell began to rely on the protective environment provided by the host cell.

Due to mitochondria's primary function in cell metabolism, mitochondria may be used for treating various disorders. There is also a need to utilize mitochondria for drug delivery and some other therapeutic and diagnostic purposes.

Weixler ("Thieme E-Jounrals - The Thoracic and Cardiovascular Surgeon," 2019-02-17, XP055970479, www.thieme-connect.de/products/ejournals/html/10.1055/s-0039-1678837) discloses preventing right heart failure in pressure-overload hypertrophy through transplantation of autologous mitochondria.

Douglas B. Cowan et al. (Plos One, 2016-08-08, vol. 11, no. 8, pages e0160889) disclose that mitochondria can be delivered to the myocardium by vascular perfusion, which can decrease myocardial infarct size and enhance post-ischemic functional recovery.

WO 2017/095940 A1 discloses preparation of chondrisomes derived from mitochondria, and related methods.

Roushandeh Amaneh Mohammadi et al. (Cytotechnology, 2019-01-31, vol. 71, no. 2, pages 647-663) disclose that mitochondrial transplantation can be a master key for treatment of variety of diseases or injuries.

Bayeva Marina et al. (Journal of The American College of Cardiology, 2012-12-05, vol. 61, no. 6, pages 599-610) disclose a review of mitochondrial biogenesis, mitochondrial oxidative stress, and mitochondrial iron handing.

Mcmurray J J et al. (The Lancet, 2005-05-28, vol. 365, no. 9474, pages 1877-1889) disclose a review of treatment of heart failure, particularly a treatment of reduced left-ventricular systolic function.

CA 3049099 A1 discloses a method for producing a novel myocardial stem cell for use in treatment and/or prevention of cardiac failure, a cell population including myocardial stem cells having activated mitochondria, a cell preparation for use in treatment and/or prevention of cardiac failure containing the myocardial stem cell or the cell population, and a liposome for use in producing the myocardial stem cell.

WO 2017/124037 A1 discloses pharmaceutical compositions comprising mitochondria and methods of treating disorders using such pharmaceutical compositions.

US 2018/340022 A1 discloses treatment of age-related conditions for example by administering an agent that inhibits the interaction of GDF11 and follistatin.

US 2007/015777 A1 discloses a method of improving the contractility of a heart comprising (a) identifying a subject or patient with aberrant or decreased contractility; and (b) treating said subject with an inhibitor of the ubiquitin proteasome pathway.

US 2009/202606 A1 discloses methods for treating or preventing cardiac conditions in a subject comprising administering to the subject two or more isoforms of hepatocyte growth factor (HGF).

Shin et al. (JACC: Basic to Translational Science, 2019-12, vol. 4, no. 8, pages 871-888) disclose that intracoronary delivery of mitochondria is safe and efficacious therapy for myocardial ischemia-reperfusion injury.

### SUMMARY

The invention is defined by the claims and any other aspects, configurations, instances or embodiments set forth herein not falling within the scope of the claims are for information only. Any references in the description to methods of treatments refer to the composition or the agent of the present invention for use in a method of treatment of the human (or animal) body by therapy. The present disclosure provides pharmaceutical compositions comprising mitochondria and such pharmaceutical compositions for use in methods of treating disorders . Described and not claimed *per se* are diagnostic and imaging methods using such pharmaceutical compositions. The described pharmaceutical compositions for use are based, at least in part, on the discovery that isolated mitochondria themselves, and isolated mitochondria linked to a therapeutic agent, diagnostic agent and/or imaging agent, can be delivered to a patient's tissue by injecting them into the patient's blood vessels. That is, direct injection or application of mitochondria to the target tissue, while contemplated by certain methods described herein, is not always necessary. Rather, in some instances, the pharmaceutical compositions for use described herein take advantage of the discovery that after mitochondria are injected or infused, for example, into an artery, the mitochondria can transverse the artery wall and be taken up by cells of the patient's tissues. The pharmaceutical compositions for use described herein can provide localized and general distribution of mitochondria or mitochondria with therapeutic agents to tissues or cells for a variety of treatment purposes using relatively simple medical procedures.

According to claim 1, provided herein, *inter alia,* is a composition comprising isolated mitochondria or a combined mitochondrial agent that comprises isolated mitochondria for use in the treatment or prevention of heart failure in a patient having pulmonary disease, wherein said composition is to be administered in a therapeutically effective amount to the patient, and wherein the patient has or is at risk of developing heart failure-right ventricular hypertrophy (RVH) or right ventricular failure (RVF). In some embodiments, the composition is administered to the subject by intramyocardial injection. In some embodiments, the pulmonary disease affects right ventricular function. In some embodiments, the composition is administered to the subject by injecting the composition into a blood vessel of the subject. In some embodiments, the mitochondria are autogeneic. In some embodiments, the mitochondria are allogeneic. In some embodiments, the mitochondria are xenogeneic.

According to claim 5, provided herein, *inter alia,* is a composition comprising isolated mitochondria or a combined mitochondrial agent that comprises isolated mitochondria for use in maintaining right ventricular (RV) contractility, maintaining RV capillary density, preventing RV dilatation, or delaying the onset of RVF in a patient, wherein said composition is to be administered in a therapeutically effective amount to the patient, wherein the patient has a pulmonary disease and has or is at risk of developing heart failure-right ventricular hypertrophy (RVH) or right ventricular failure (RVF), and wherein said pulmonary disease affects right ventricular function. In some embodiments, the composition is administered to the subject by intramyocardial injection. In some embodiments, the composition is administered to the subject by injecting the composition into a blood vessel of the subject. In some embodiments, the mitochondria are autogeneic. In some embodiments, the mitochondria are allogeneic. In some embodiments, the mitochondria are xenogeneic.

Described and not claimed *per se* are methods of maintaining left ventricular (LV) contractility, maintaining LV capillary density, preventing LV dilatation, or delaying the onset of left ventricular failure (LVF) in a subject, the method comprising administering to the subject a therapeutically effective amount of a composition comprising isolated mitochondria or a combined mitochondrial agent. The composition can be administered to the subject by intramyocardial injection. The subject may have or may be at risk of developing heart failure-right ventricular hypertrophy (RVH), left ventricular hypertrophy (LVH), right ventricular failure (RVF), or left ventricular failure (LVF). The subject may have a pulmonary disease. The pulmonary disease may affect left ventricular function. The composition may be administered to the subject by injecting the composition into a blood vessel of the subject. The mitochondria may be autogeneic. The mitochondria may be allogeneic. The mitochondria may bexenogeneic.

Described and not claimed *per se* are methods of maintaining ventricular contractility in a subject, the method comprising
identifying the subject in need thereof; and
administering to the subject a therapeutically effective amount of a composition comprising isolated mitochondria or a combined mitochondrial agent. The subject may be identified by measuring end-systolic pressure-volume (ESPV).

Described and not claimed *per se* are methods maintaining ventricular capillary density in a subject, the method comprising
identifying the subject in need thereof; and
administering to the subject a therapeutically effective amount of a composition comprising isolated mitochondria or a combined mitochondrial agent.

Described and not claimed *per se* are methods of reducing the risk of ventricular dilatation in a subject, the method comprising
identifying the subject in need thereof; and
administering to the subject a therapeutically effective amount of a composition comprising isolated mitochondria or a combined mitochondrial agent.

In some embodiments, the subject is identified as having diabetes, obesity, hypertension, alcohol abuse, cocaine use and abuse, bacteria infection, virus infection, fungi infection, parasite infection, exposure to toxins (e.g., lead, mercury or cobalt), arrhythmias, or late-stage pregnancy complication.

Described and not claimed *per se* are methods of delaying the onset of heart failure in a subject, the method comprising
identifying the subject in need thereof; and
administering to the subject a therapeutically effective amount of a composition comprising isolated mitochondria or a combined mitochondrial agent. The subject may be identified as having right ventricular hypertrophy or left ventricular hypertrophy.

Described and not claimed *per se* are methods of treating heart failure, delaying the onset of heart failure, reducing the risk of developing heart failure in a subject, the method comprising administering to the subject a therapeutically effective amount of a composition comprising isolated mitochondria or a combined mitochondrial agent. The composition can be administered to the subject by intramyocardial injection. The method may comprise identifying the subject as having a risk of developing heart failure. The subject may have a pulmonary disease. The composition can be administered to the subject by injecting the composition into a blood vessel to the heart.

According to claim 6, provided herein, *inter alia,* is a composition comprising isolated mitochondria or a combined mitochondrial agent that comprises isolated mitochondria for use in the treatment of heart hypertrophy, delaying of the onset of heart hypertrophy, reduction of the risk of developing heart hypertrophy in a patient, wherein said composition is to be administered in a therapeutically effective amount to the patient, wherein the patient has a pulmonary disease and wherein the patient has or is at risk of developing RVH or RVF.. In some embodiments, the composition is administered to the subject by intramyocardial injection. In some embodiments, the method comprises identifying the subject as having a risk of developing heart hypertrophy. In some embodiments, the composition is administered to the subject by injecting the composition into a blood vessel to the heart.

In certain embodiments, the blood vessel is the blood vessel or part of the vascular system which carries the blood to the target site, the target organ, or the target area, e.g., the coronary artery of the subject, the hepatic portal vein of the subject, the greater pancreatic artery of the subject, or the prostate artery of the subject.

In certain embodiments, the mitochondria can have different sources, e.g., the mitochondria can be autogeneic, allogeneic, or xenogeneic. In certain embodiments, the autogeneic mitochondria can have exogenous mtDNA. In some embodiments, the mitochondria are from a subject's first-degree relative.

In some embodiments, the described composition or agent for use includes the step of collecting the isolated mitochondria from cells prior to administration. The isolated mitochondria or combined mitochondrial agent can be administered to the subject immediately after the isolated mitochondria are collected from cells.

In one aspect, the disclosure provides compositions comprising isolated mitochondria or a combined mitochondrial agent for use, in combination with a carrier. In some embodiments, the composition is a pharmaceutical composition. The carrier can be any suitable carrier, e.g., respiration buffer, mitochondria buffer, sterile mitochondria buffer, University of Wisconsin (UW) solution, blood, serum, or a contrast agent.

In all compositions for use described herein, the combined mitochondrial agent can comprise a pharmaceutical agent. The pharmaceutical agent can be a therapeutic agent. Described and not claimed *per se* is that the imaging agent can be radioactive. Described and not claimed *per se* is that the imaging agent is ¹⁸F-Rhodamine 6G, or iron oxide nanoparticle. In some embodiments, the pharmaceutical agent is linked to mitochondria by a covalent bond. Alternatively, or in addition, the pharmaceutical agent is embedded in the mitochondria. A combined mitochondrial agent can include an antibody or an antigen binding fragment. Furthermore, in all compositions for use described herein, the mitochondria can be autogeneic, allogeneic, or xenogeneic. In some embodiments, the mitochondria have exogenous DNA (e.g., mtDNA).

As used herein, the term "isolated mitochondria" means functional and intact mitochondria that are free of extraneous eukaryotic cell material.

A "combined mitochondrial agent" is an isolated mitochondrion that is combined artificially with a pharmaceutical, diagnostic, or imaging, or any other agent. The agent is combined with a mitochondrion in any fashion, for example, linked (e.g., chemically or electrostatically linked) to a mitochondrion, attached to a mitochondrion, embedded in the mitochondrial membrane, substantially enclosed within a mitochondrion, or encapsulated entirely by a mitochondrion, as long as the mitochondrion and the agent are in physical contact with each other. Combined mitochondrial agents are designed such that the mitochondrion act as a "carrier" that can transport the agent to a patient's tissues after injection.

The terms "subject" and "patient" are used throughout the specification to describe an animal, human or non-human, to whom treatment according to the methods of the present disclosure is provided. Veterinary applications are clearly anticipated by the present disclosure. The term includes but is not limited to birds, reptiles, amphibians, and mammals, e.g., humans, other primates, pigs, rodents such as mice and rats, rabbits, guinea pigs, hamsters, cows, horses, cats, dogs, sheep and goats. Preferred subjects are humans, farm animals, and domestic pets such as cats and dogs.

The term "treat(ment)," is used herein to denote delaying the onset of, inhibiting, alleviating the effects of, or prolonging the life of a patient suffering from, a condition, e.g., a disease described herein.

An "ischemia-related disease" is a disease that involves ischemia. Ischemia, as used herein, is a reduced blood flow to an organ and/or tissue. The reduced blood flow may be caused by any suitable mechanism, including a partial or complete blockage (an obstruction), a narrowing (a constriction), and/or a leak/rupture, among others, of one or more blood vessels that supply blood to the organ and/or tissue.

By "immediately after mitochondria are collected from cells" is meant immediately after mitochondria are collected from cells and before any substantial reduction in viability of the mitochondria can occur.

As used herein, the term "transplantation" is used throughout the specification as a general term to describe the process of implanting an organ, tissue, mass of cells, individual cells, or cell organelles into a recipient. The term "cell transplantation" is used throughout the specification as a general term to describe the process of transferring at least one cell, e.g., an islet cell, or a stem cell, to a recipient. For example, such transplantation can be performed by removing the β-cells (or intact islets) from a donor's pancreas and putting them into a recipient patient whose pancreas cannot produce sufficient insulin. The terms include all categories of transplants known in the art, except blood transfusions. Transplants are categorized by site and genetic relationship between donor and recipient. The term includes, e.g., autotransplantation (removal and transfer of cells or tissue from one location on a patient to the same or another location on the same subject), allotransplantation (transplantation between members of the same species), and xenotransplantation (transplantations between members of different species).

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

FIG. 1 is a schematic diagram depicting a method of mitochondria isolation.

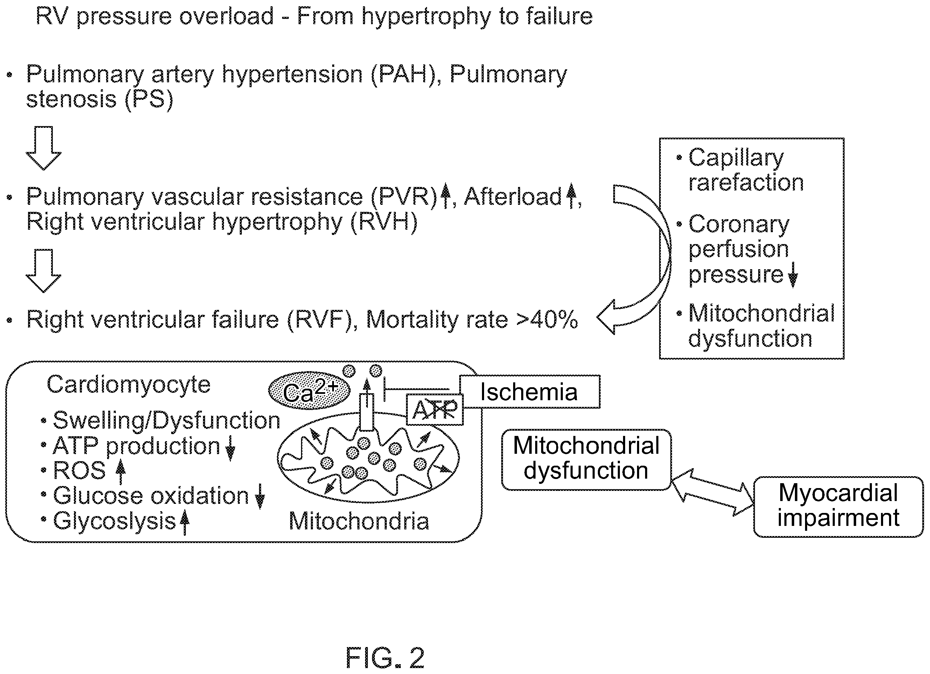

FIG. 2 is a schematic diagram depicting disease outcomes associated with ventricular overload.

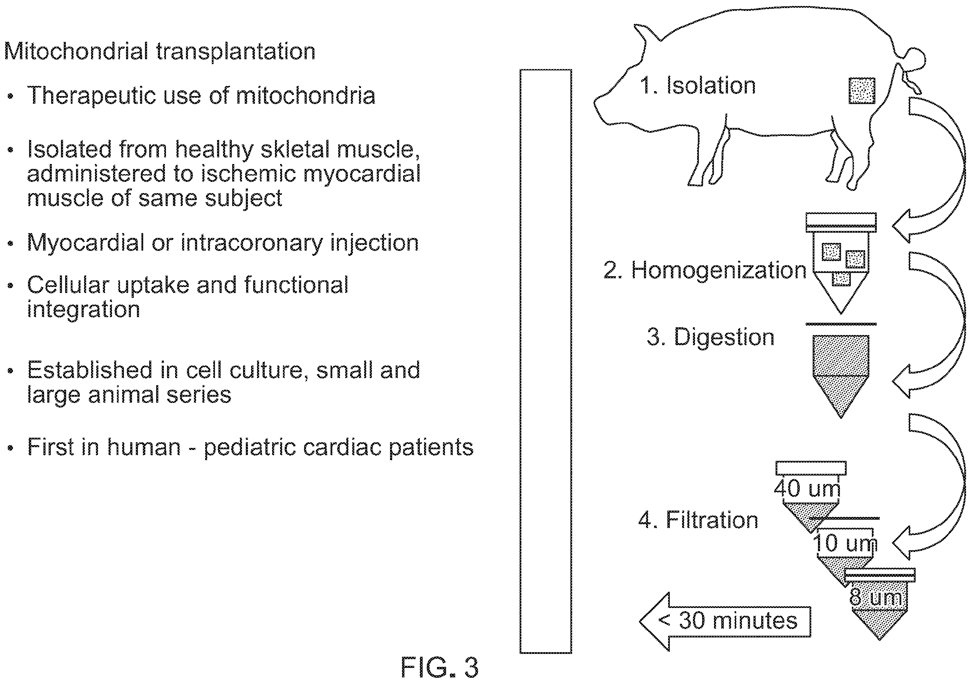

FIG. 3 is a schematic diagram depicting a method overview of mitochondrial transplantation in a subject.

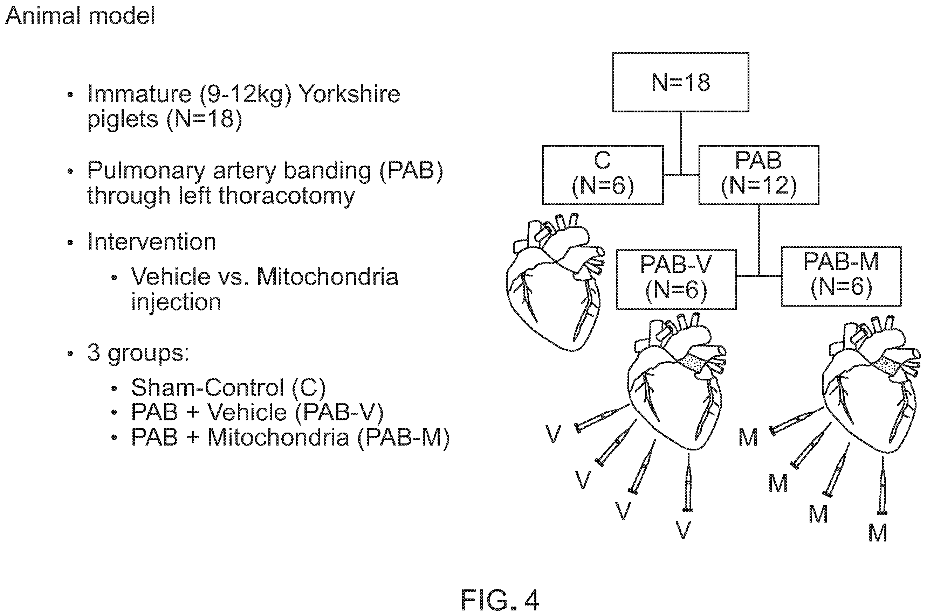

FIG. 4 is a schematic diagram depicting an animal model study utilizing pulmonary artery banding (PAB).

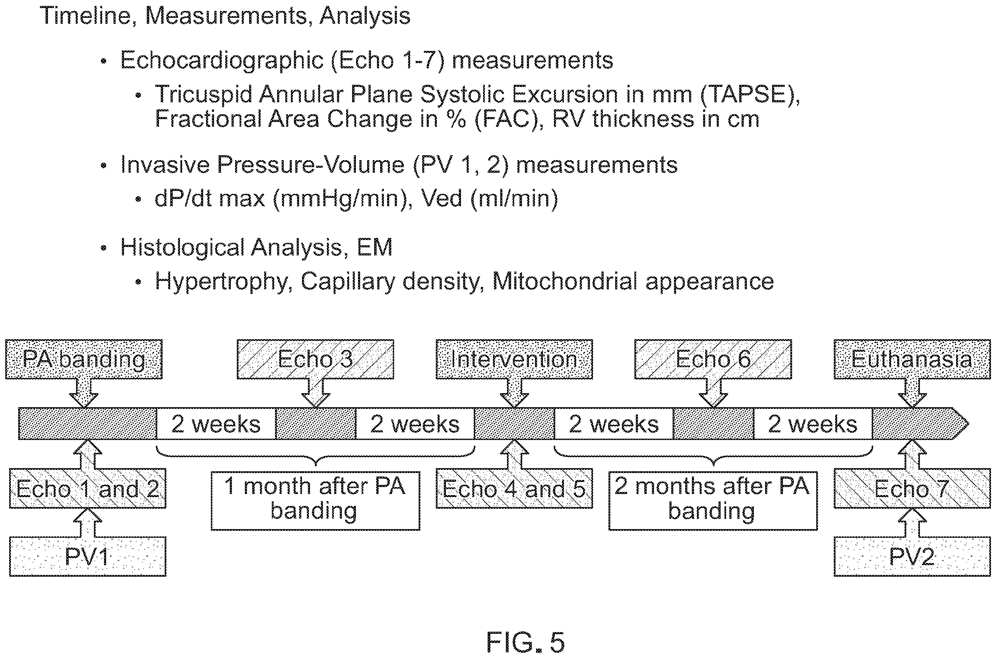

FIG. 5 is a schematic diagram depicting a timeline of measurements and analysis for an experiment.
FIG. 6 is a line graph showing functional area change (FAC), in percentage, at baseline, 1 month after PAB, and at euthanasia for the control (C, also called "sham" group), PAB-V (Vehicle), and PAB-M (Mitochondria) groups.
FIG. 7 is a line graph showing tricuspid annular plane systolic excursion (TAPSE), in mm, at baseline, 1 month after PAB, and at euthanasia for the control (C, also called "sham" group), PAB-V (Vehicle), and PAB-M (Mitochondria) groups.
FIG. 8 is a line graph showing Right Ventricular (RV) wall thickness, in cm, at baseline, 1 month after PAB, and at euthanasia for the control (C, also called "sham" group), PAB-V (Vehicle), and PAB-M (Mitochondria) groups.
FIG. 9 is a box plot showing dP/dt max, in mmHg/sec, at euthanasia for the control (C, also called "sham" group), PAB-V (Vehicle), and PAB-M (Mitochondria) groups.
FIG. 10 is a line graph showing dP/dt max, in mmHg/sec, at baseline and at euthanasia for the control (C, also called "sham" group), PAB-V (Vehicle), and PAB-M (Mitochondria) groups.
FIG. 11A is an immunofluorescence image showing TUNEL staining to illuminate apoptotic cells (white arrows) in the TUNEL-positive control group. Cardiomyocytes are stained by desmin staining, and nuclei are stained by DAPI staining.
FIG. 11B is an immunofluorescence image showing TUNEL staining to illuminate apoptotic cells (white arrows) in the control/sham group. Cardiomyocytes are stained by desmin staining, and nuclei are stained by DAPI staining.
FIG. 11C is an immunofluorescence image showing TUNEL staining to illuminate apoptotic cells (white arrows) in the PAB-V group. Cardiomyocytes are stained by desmin staining, and nuclei are stained by DAPI staining.
FIG. 11D is an immunofluorescence image showing TUNEL staining to illuminate apoptotic cells (white arrows) in the PAB-M group. Cardiomyocytes are stained by desmin staining, and nuclei are stained by DAPI staining.
FIG. 11E is a bar graph showing the ratio of % desmin per field of vision/ number of nuclei per field of vision (P<0.01**).
FIG. 12A is an immunofluorescence image showing CD31 staining to illuminate capillary density (white arrows) in the control/sham group. Cardiomyocytes are stained via desmin staining, and nuclei are stained via DAPI staining.
FIG. 12B is an immunofluorescence image showing CD31 staining to illuminate capillary density (white arrows) in the PAB-V group. Cardiomyocytes are stained via desmin staining, and nuclei are stained via DAPI staining.
FIG. 12C is an immunofluorescence image showing CD31 staining to illuminate capillary density (white arrows) in the PAB-M group. Cardiomyocytes are stained via desmin staining, and nuclei are stained via DAPI staining.
FIG. 13A is electron microscopy showing the number and shape of mitochondria in the control/sham group.
FIG. 13B is electron microscopy showing the number and shape of mitochondria in the PAB-V group.
FIG. 13C is electron microscopy showing the number and shape of mitochondria in the PAB-C group.

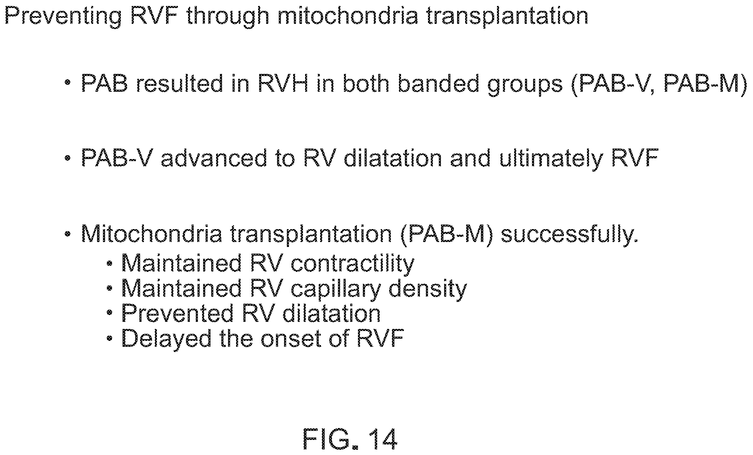

FIG. 14 is a schematic depicting disease results and outcomes associated with mitochondrial transplantation treatment.
FIG. 15 is an immunofluorescence image showing pictures of control, RV hypertrophy (RVH), and RVH with mitochondrial transplantation.
FIG. 16 is a box plot showing ATP levels in cardiomyocytes in control, untreated hypertrophied cardiomyocytes (H no mito), and mitochondria-treated hypertrophied cardiomyocytes (H heart mito, H gastro mito, and H soleus mito), *p=0.05 versus control, #p=0.001 versus untreated hypertrophied cardiomyocytes (H no mito).
FIG. 17A is an immunofluorescence image showing TUNEL staining to illuminate apoptotic cells (white arrows) in the control/sham, PAB-V, and PAB-M groups. Cardiomyocytes are stained via desmin staining, and nuclei are stained via DAPI staining.
FIG. 17B is a box plot showing TUNEL positive nuclei per 1000 nuclei, (*p=0.01 versus control and #p=0.05 PAB-V versus PAB-M.
FIG. 17C is microscopy showing representative histological sections to detect fibrosis in the control/sham, PAB-V, and PAB-M groups.
FIG. 17D is a box plot showing ratio of % fibrosis per field of vision at study endpoint (*p=0.01 versus control and #p=0.05 PAB-V versus PAB-M).
FIG. 18A is a box plot showing RV wall thickness, in cm, at baseline for the control (C, also called "sham"), PAB-V, and PAB-M groups.
FIG. 18B is a box plot showing RV wall thickness, in cm, at 1 month after PAB for the control (C, also called "sham"), PAB-V, and PAB-M groups.
FIG. 18C is a box plot showing RV wall thickness, in cm, at euthanasia for the control (C, also called "sham"), PAB-V, and PAB-M groups.
FIG. 19A is a box plot showing functional area change (FAC), in percentage, at baseline for the control (C, also called "sham"), PAB-V, and PAB-M groups.
FIG. 19B is a box plot showing functional area change (FAC), in percentage, at 1 month after PAB for the control (C, also called "sham"), PAB-V, and PAB-M groups.
FIG. 19C is a box plot showing functional area change (FAC), in percentage, at euthanasia for the control (C, also called "sham"), PAB-V, and PAB-M groups.
FIG. 20A is a box plot showing tricuspid annular plane systolic excursion (TAPSE), in mm, at baseline for the control (C, also called "sham"), PAB-V, and PAB-M groups.
FIG. 20B is a box plot showing tricuspid annular plane systolic excursion (TAPSE), in mm, at 1 month after PAB for the control (C, also called "sham"), PAB-V, and PAB-M groups.
FIG. 20C is a box plot showing tricuspid annular plane systolic excursion (TAPSE), in mm, at euthanasia for the control (C, also called "sham"), PAB-V, and PAB-M groups.
FIG. 21A is a box plot showing dP/dt max, in mmHg/sec, at baseline for the control (C, also called "sham"), PAB-V, and PAB-M groups.
FIG. 21B is a box plot showing dP/dt max, in mmHg/sec, at euthanasia for the control (C, also called "sham"), PAB-V, and PAB-M groups.

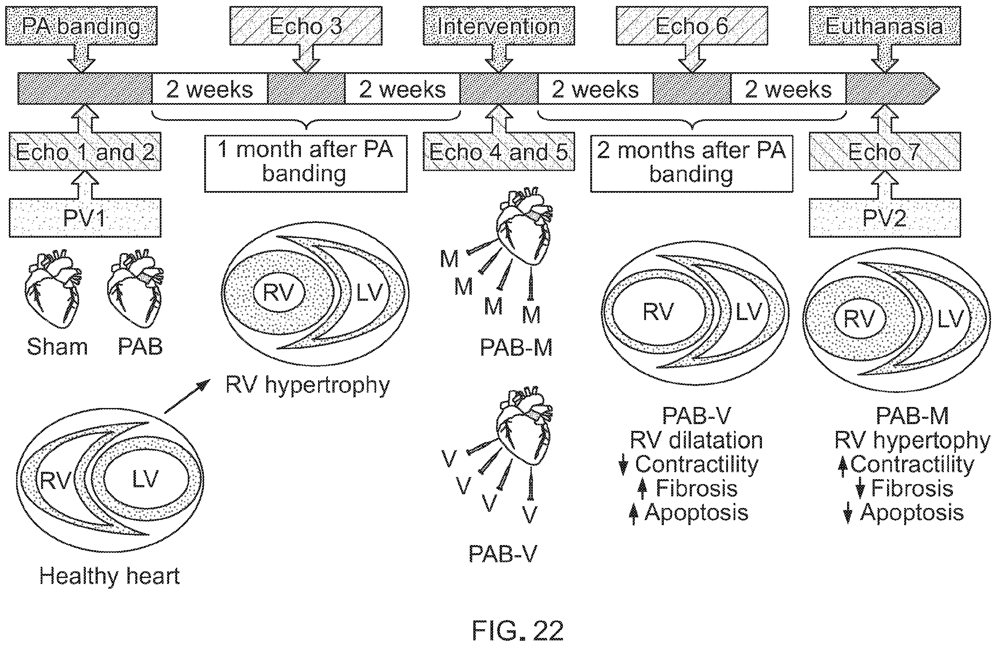

FIG. 22 is a schematic diagram showing an animal model study utilizing pulmonary artery banding (PAB) and a summary of some of the clinical findings.

### DETAILED DESCRIPTION

Right ventricular hypertrophy (RVH) and failure (RVF) are major causes of cardiac morbidity and mortality affecting the long-term outcome in patients where the right ventricle (RV) is abnormally loaded due to pulmonary hypertension, outflow tract obstruction, or is functioning as the systemic ventricle. As an initial compensatory step, the RV adapts to these hemodynamic changes by increasing wall thickness to provide higher contractility to overcome the increase in afterload. Ultimately, these mechanisms are not sufficient and hypertrophy proceeds to dilation and contractile failure. Clinical observation indicates that these compensatory changes preserve contractile function more effectively and for longer periods of time on the left side than on right side, where failure occurs more rapidly. Mitochondrial function directly affects cardiac function and contractility. The lack of adaptation of the RV to increased pressure-loading long-term is associated with the inability of mitochondrial and calcium handling mechanisms to keep up with demands from thickening cardiac muscle tissue. Futile calcium cycling with adenosine-tri-phosphate (ATP) expenditure and electron transport chain (ETC) dysfunction further limiting ATP synthesis, lead to bio-energetic failure (McCully JD, Rousou AJ, Parker RA, Levitsky S. Age and gender differences in mitochondrial oxygen consumption and free matrix calcium during ischemia/reperfusion and with cardioplegia and diazoxide. Ann Thorac Surg. 2007;83:1102-1109). These events eventually overwhelm cellular regulatory mechanisms and lead to the more rapid worsening of cardiac function in RVH.

Mitochondrial enzyme activity as well as mitochondrial DNA (mtDNA) content progressively decrease from hypertrophy to failure and therefore, play a significant role in RV dysfunction. Corresponding with these findings, the present disclosure has demonstrated using combined microarray and proteomic analysis of matched samples that mitochondrial function with regards to mitochondrial quantity/mass are equally as important as cardiac muscle tissue development in adaptation of the thin-walled RV to pathological loading. In addition, activation of proapoptotic pathways, particularly related to mitochondria and a downregulation of calcium signaling pathways have been associated with progression to failure. An initial upregulation of oxidative phosphorylation and associated calcium handling for mitochondrial stabilization is in response to meet energy demands of cardiac muscle growth adapting the thin walled RV to pressure overload. However, with prolonged exposure to increased pressure loading, mitochondria are unable to adapt resulting in rapid deterioration with decline in contractile function. Progression to heart failure is associated with a decline in energy reserve capacity and compensatory mechanisms can no longer support the imbalance of decreasing energy supply and increasing demand of adaptive RV wall thickening.

The central role of mitochondria in the progression of hypertrophy to heart failure has been recognized. The current therapy for patients with right heart failure is largely limited to treatments targeting lung function rather than directly interfering in the critical myocardial energetic deficit as a result of mitochondrial dysfunction. Previous studies demonstrated the successful and safe technique of therapeutic transplantation of autologous respiration-competent mitochondria, which replace and/or replenish the pool of native, injured mitochondria with viable mitochondria isolated from healthy tissue. However, therapeutic success has mainly been demonstrated in models of acute ischemia-reperfusion injury where the beneficial effect of mitochondrial transplantation was established (McCully JD, Cowan DB, Pacak CA, Toumpoulis IK, Dayalan H, Levitsky S. Injection of isolated mitochondria during early reperfusion for cardioprotection. Am J Physiol Heart Circ Physiol. 2009;296(1):H94-105). Less is known whether long-term mitochondrial dysfunction in addition to adaptive maintenance of mitochondrial function due to progressive pressure-overload hypertrophy can be achieved with mitochondrial transplantation. Also, with a combination of metabolic adaptive alterations and mitochondrial dysfunction, the source of mitochondria for transplantation might play a critical role. As it has been reported, mitochondria adapt to their role demanded by the tissue that they are supplying (Fernández-Vizarra E, Enríquez JA, Pérez-Martos A, Montoya J, Fernández-Silva P. Tissue-specific differences in mitochondrial activity and biogenesis. Mitochondrion. 2011;11(1):207-213.). Mitochondria of fast-twitch skeletal muscle compared to slow-twitch skeletal muscle are accustomed to highly glucose metabolizing cells which can rapidly adapt to increased energy demands. It has been established that hypertrophied and failing myocardium switch to the use of glucose as metabolic substrate (Doenst T, Nguyen TD, Abel ED. Cardiac metabolism in heart failure: Implications beyond ATP production. Circ Res. 2013;113:709-724.). Thus, the source of mitochondria used for transplantation might be of relevance since the goal is to restore defective energy production and mitochondrial dynamics in the failing heart. With already impaired cardiac mitochondria in the failing heart, mitochondria from other cell sources have to be harvested for transplantation.

The present disclosure is based in part on the surprising discovery that mitochondria can be used to prevent, treat, and/or reduce one or more of the symptoms of heart failure, even before the heart failure has occurred. Thus, described and not claimed *per se* are methods of minimizing heart failure, reducing risk of heart failure, ameliorating at least one symptom of heart failure, preventing or treating cell damage, tissue damage, and/or organ damage associated with heart failure, in a subject at risk of heart failure.

According to claim 1, one aspect of present disclosure provides a composition comprising isolated mitochondria or a combined mitochondrial agent that comprises isolated mitochondria for use in the treatment or prevention of heart failure in a patient having pulmonary disease, wherein said composition is to be administered in a therapeutically effective amount to the patient, and wherein the patient has or is at risk of developing heart failure-right ventricular hypertrophy (RVH) or right ventricular failure (RVF). In some embodiments, the composition is administered to the subject by direct injection, intramyocardial injection, or infusion.

The present disclosure is also based, at least in part, on the discovery that isolated mitochondria, and isolated mitochondria linked to a therapeutic agent, diagnostic agent and/or imaging agent, can be delivered to a patient's tissue by injecting them into the patient's blood vessels. Skilled practitioners can locally and/or generally distribute mitochondria to tissues and/or cells of a patient for a variety of purposes, using relatively simple medical procedures. Further, mitochondria can be used as carrier agents, e.g., to deliver therapeutic, diagnostic, and/or imaging agents, to a patient's tissues. Compared to some traditional therapeutic regimens that involve nanoparticles, it is further noted that mitochondria are not toxic and do not cause any substantial adverse immune or auto-immune response.

While not intending to be bound by any theory, it is believed that infused mitochondria extravasate through the capillary wall by first adhering to the endothelium. After they are injected or infused into an artery, mitochondria can cross the endothelium of the blood vessels and be taken up by tissue cells through an endosomal actin-dependent internalization process.

### Combined Mitochondrial Agents

Combined mitochondrial agents include mitochondria that are physically associated with an agent, such as a therapeutic agent, a diagnostic agent, and/or an imaging agent.

A therapeutic agent can be any agent that has a therapeutic or prophylactic use. Exemplary therapeutic agents include, e.g., therapeutic agents for ischemia-related disorders, cytotoxic agents for treating cancer, among many others. In some instances, mitochondria can deliver therapeutic agents to specific cells, for example, tumor cells. The therapeutic agent may be, e.g., an intracellular inhibitor, deactivator, toxin, arresting substance and/or cytostatic/cytotoxic substance that, once inside a cell, inhibits, destroys, arrests, modifies and/or alters the cell such that it can no longer function normally and/or survive. The therapeutic agent can be an agent to restore a cell's proper function, for example, a DNA vector for gene therapy. A therapeutic agent can be, e.g., an inorganic or organic compound; a small molecule (less than 500 daltons) or a large molecule; a proteinaceous molecule, such as a peptide, polypeptide, protein, post-translationally modified protein, or antibody; or a nucleic acid molecule, such as a double-stranded DNA, single-stranded DNA, double-stranded RNA, single-stranded RNA, or a triple helix nucleic acid molecule. In some embodiments, a therapeutic agent can be a natural product derived from any known organism (e.g., from an animal, plant, bacterium, fungus, protist, or virus) or from a library of synthetic molecules. In some embodiments, a therapeutic agent can be a monomeric or a polymeric compound. Some exemplary therapeutic agents include cytotoxic agents, DNA vectors, small interfering RNAs (siRNA), micro RNAs (miRNA), reactive peptides, nanoparticles, microspheres, and fluorescent molecules.

A diagnostic agent is an agent that has diagnostic use. As mitochondria can carry a diagnostic agent into a cell, the diagnostic agent can be designed to determine the condition within a cell, for example pH and oxidative stress within a cell.

An imaging agent is an agent that is employed for use in imaging techniques. The techniques or modalities include, but are not limited to, X-rays, computed tomography (CT), magnetic resonance imaging (MRI), scintigraphy, fluorescence, ultrasound, etc. The imaging agent can be florescent and/or radioactive. An imaging agent can also be a diagnostic agent. Exemplary imaging agents include, but are not limited to, MitoTracker fluorophores (Thermo Fisher Scientific Inc.), CellLight^{®} RFP, BacMam 2.0 (Thermo Fisher Scientific Inc.), pH-sensitive pHrodo fluorescent dyes (Thermo Fisher Scientific Inc.), ¹⁸F-Rhodamine 6G, ¹⁸F-labeled rhodamine B, magnetic iron oxide nanoparticles, and gold- and platinum-based nanoparticles.

As discussed above, a combined mitochondrial agent comprises a mitochondria and an agent that are in direct and/or indirect physical contact with each other. For example, an agent can be linked to mitochondria, attached to mitochondria, embedded in the mitochondrial membrane, or completely or partially enclosed in mitochondria. In some instances, a pharmaceutical agent can be linked to mitochondria covalently. In some instances, the agent is linked to constituents of mitochondrial membrane directly through a covalent bond (e.g., a carboxamide bond and a disulfide bond), or indirectly through a linker (e.g., a peptide linker) or another covalently bonded agent. In other instances, an agent can be linked to mitochondria non-covalently, for example, through hydrophobic interaction, Van der Waals interaction, and/or electrostatic interaction, etc.

In some embodiments, a combined mitochondrial agent can comprise two or more different types of agents, for example, two different kinds of therapeutic agents, three different kinds of imaging agents, one therapeutic agent and one imaging agent, a therapeutic agent and a diagnostic agent, etc. Skilled practitioner will appreciate that any variation is possible.

One particularly useful linker to link mitochondria and an agent provides a sustained release of the agent upon injection. This can be accomplished, for example, using a hydrazone functional group. For example, a hydrazone is formed to covalently bind an agent to constituents on the mitochondrial membrane. Once this combined mitochondrial agent is taken up by cells, the change in pH will result in hydrolysis of the hydrazone, releasing the bound agent inside the cell.

In some embodiments, a therapeutic agent can be linked to the outer mitochondrial membrane using functionalized surface chemistry. In some cases, heterobifunctional chemistries can link a therapeutic agent to the mitochondrial surface, and once they are internalized, these agents can be released through interactions with intercellular esterases (e.g. via interaction with an acetoxymethyl ester). The UV-light activation and Near-Infrared light activation strategies are described, e.g., in Zhou, Fang, Hanjie Wang, and Jin Chang, "Progress in the Field of Constructing Near-Infrared Light-Responsive Drug Delivery Platforms," Journal of Nanoscience and Nanotechnology 16.3 (2016): 2111-2125; Bansal, Akshaya, and Yong Zhang, "Photocontrolled nanoparticle delivery systems for biomedical applications," Accounts of chemical research 47.10 (2014): 3052-3060; Barhoumi, Aoune, Qian Liu, and Daniel S. Kohane, "Ultraviolet light-mediated drug delivery: Principles, applications, and challenges," Journal of Controlled Release 219 (2015): 31-42.

### Pharmaceutical and Other Compositions

The disclosure provides compositions that comprise isolated mitochondria for use, or combined mitochondrial agents for use.

A pharmaceutical composition for use described herein may include mitochondria and/or combined mitochondria agents and a pharmaceutically acceptable carrier. As used herein, the language "pharmaceutically acceptable carrier" includes saline, solvents, dispersion media, coatings, antibacterial and antifungal agents, isotonic and absorption delaying agents, and the like, compatible with pharmaceutical administration. In some embodiments, the pharmaceutically acceptable carrier is phosphate buffered saline, saline, Krebs buffer, Tyrode's solution, contrast media, or omnipaque, or a mixture thereof. In some embodiments, the pharmaceutically acceptable carrier is sterile mitochondria buffer (300 mM sucrose; 10 mM K+-HEPES (potassium buffered (4-(2-hydroxyethyl)-1-piperazineethanesulfonic acid, pH 7.2); 1 mM K+-EGTA, (potassium buffered ethylene glycol tetraacetic acid, pH 8.0)). In some embodiments, the pharmaceutically acceptable carrier is respiration buffer (250 mM sucrose, 2 mM KH₂PO₄, 10 mM MgCl₂, 20 mM K-HEPES Buffer (pH 7.2), and 0.5 mM K-EGTA (pH 8.0)).

Pharmaceutical compositions are typically formulated to be compatible with its intended route of administration. Examples of routes of administration include parenteral, e.g., intravenous, intradermal, subcutaneous, oral (e.g., inhalation), sublingual, transdermal (e.g., topical), transmucosal, and rectal administration.

A pharmaceutical composition can be formulated for various clinical uses, e.g., imaging, treating wounds, treating injuries, preserving organs, improving mitochondrial functions in organs or tissues, and skin care. In some cases, the pharmaceutically acceptable carrier is a contrast agent for imaging purpose. In some embodiments, the pharmaceutical composition may include antiseptic agents, antibacterial agents (e.g., antibiotics), antifungal agents, disinfectants, analgesic agents, anesthetic agents, steroids, nutritional supplements, ethereal oils, etc. An anesthetic agent is a drug that can prevent pain during surgery or treatment. Exemplary analgesic agents include, without limitation, paracetamol, nonsteroid anti-inflammatory drugs, salicylates, ibuprofen and lidocaine. Exemplary antibacterial agents include, without limitation, dichlorobenzyl alcohol, amylmetacresol and antibiotics. Exemplary antibiotics include penicillins carbapenems, cephalosporins aminoglycosides, bacitracin, gramicidin, mupirocin, chloramphenicol, thiamphenicol, lincomycin, clindamycin, macrolides, novobiocin, polymyxins, rifamycins, spectinomycin, tetracyclines, vancomycin, teicoplanin, streptogramins, anti- folate agents, sulfonamides, trimethoprim, pyrimethamine, nitrofurans, methenamine mandelate, methenamine hippurate, nitroimidazoles, quinolones, fluoroquinolones, isoniazid, ethambutol, pyrazinamide, para-aminosalicylic acid, cycloserine, capreomycin, ethionamide, prothionamide, thiacetazone and viomycin. Antiseptic agents are antimicrobial substances that can be applied to living tissue/skin to reduce the possibility of infection, sepsis, or putrefaction. Exemplary antiseptics include, without limitation, chlorhexidine and salts thereof, benzalkonium and salts thereof, triclosan and cetylpyridium chloride. Exemplary antifungal agents include, without limitation, tolnaftate, miconazole, fluconazole, clotrimazole, econazole, ketoconazole, itraconazole, terbinafine, amphotericin, nystatin and natamycin. Exemplary steroids include, without limitation, prednisone acetate, prednisone valerate, prednisolone, alclometasone dipropionate, fluocinolone acetonide, dexamethasone, methylprednisolone, desonide, pivolate, clocortolone pivolate, triamcinolone acetonide, prednicarbate, fluticasone propionate, flurandrenolide, mometasone furoate, desoximetasone, betamethasone, betamethasone dipropionate, betamethasone valerate, betamethasone propionate, betamethasone benzoate, diflorasone diacetate, fluocinonide, halcinonide, amcinonide, halobetasol propionate, and clobetasol propionate. Exemplary nutritional supplements include, without limitation, vitamins, minerals, herbal products and amino acids. Vitamins include without limitation, vitamin A, those in the vitamin B family, vitamin C, those in the vitamin D family, vitamin E and vitamin K. Ethereal oils include without limitation, those derived from mint, sage, fir, lavender, basil, lemon, juniper, rosemary, eucalyptus, marigold, chamomile, orange and the like. Many of these agents are described, e.g., in WO 2008152626.

Compositions for use comprising mitochondria and/or combined mitochondrial agents can be formulated in any form, e.g., liquids, semi-solids, or solids. Exemplary compositions include liquids, creams, ointments, salves, oils, emulsions, liposome formulations, among others.

### Methods of Making Compositions Comprising Mitochondria and/or Combined Mitochondrial Agents

### Isolating mitochondria

Mitochondria for use can be isolated or provided from any source, e.g., isolated from cultured cells or tissues. Exemplary cells include, but are not limited to, muscle tissue cells, cardiac fibroblasts, cultured cells, HeLa cells, prostate cancer cells, yeast, among others, and any mixture thereof. Exemplary tissues include, but are not limited to, liver tissue, skeletal muscle, heart, brain, and adipose tissue. Mitochondria can be isolated from cells of an autogenous source, an allogeneic source, and/or a xenogeneic source. In some instances, mitochondria are isolated from cells with a genetic modification, e.g., cells with modified mtDNA or modified nuclear DNA.

Mitochondria can be isolated from cells or tissues by any means known to those of skill in the art. In one example, tissue samples or cell samples are collected and then homogenized. Following homogenization, mitochondria are isolated by repetitive centrifugation. Alternatively, the cell homogenate can be filtered through nylon mesh filters. Typical methods of isolating mitochondria are described, for example, in McCully JD, Cowan DB, Pacak CA, Toumpoulis IK, Dayalan H and Levitsky S, Injection of isolated mitochondria during early reperfusion for cardioprotection, Am J Physiol 296, H94-H105. PMC2637784 (2009); Frezza, C., Cipolat, S., & Scorrano, L, Organelle isolation: functional mitochondria from mouse liver, muscle and cultured filroblasts. Nature protocols, 2(2), 287-295 (2007); and a PCT application entitled "Products and Methods to Isolate Mitochondria" (PCT/US2015/035584; WO 2015192020).

### Methods of Making Combined Mitochondrial Agents

Skilled practitioners will appreciate that an agent can be linked to mitochondria in any number of ways, e.g., by attaching to mitochondria, embedding partially or completely in the mitochondrial membrane, enclosing in mitochondria, or encapsulating within the mitochondria.

While not intending to be bound by any theory or any particular approach, it is believed that the outer membrane of mitochondria is adherent and thus particularly amenable to combination with various agents. In some embodiments, pharmaceutical agents can be attached to the outer membrane of mitochondria simply by incubation. For example, an effective amount of pharmaceutic agents can be fully mixed with isolated mitochondria in a buffer, e.g., respiration buffer, at a temperature favorable to isolated mitochondria, e.g., from 0 °C to 26 °C, from 0 °C to 4 °C, or about 0 °C, 4°C, 26 °C. This procedure is useful to attach an effective amount of pharmaceutic agents (e.g., nanoparticles, DNA vectors, RNA vectors) to mitochondria.

In some embodiments, organic cations (e.g., rhodamine and tetramethylrosamine) are readily sequestered by functioning mitochondria because of the electric potential on mitochondrial membrane. Healthy mitochondrial membranes maintain a difference in electric potential between the interior and exterior of the organelle, referred to as the membrane potential. This membrane potential is a direct result of mitochondrial functional processes, and can be lost if the mitochondria are not working properly. Lipid-soluble cations are sequestered by mitochondria as a consequence of their positive charge and of their solubility in both the inner membrane lipids and the matrix aqueous space. Similarly, in some other embodiments, anions can be attached to the outer membrane of mitochondria because of its negative charge. To link mitochondria with these pharmaceutical agents, an effective amount of pharmaceutic agents should be fully mixed with isolated mitochondria in a buffer, e.g., respiration buffer, at a temperature favorable to isolated mitochondria, e.g., about 0°C or 4°C.

The therapeutic, diagnostic, and/or imaging agent can be linked to phospholipids, peptides, or proteins on the mitochondrial membrane through a chemical bond. For example, molecules including fluorophores (pHrodo Red (Thermo Fisher Scientific, Inc.)) and metallic particles (e.g., 30 nm magnetic iron oxide nanoparticles (Sigma)) can be covalently linked to exposed amine groups on proteins and peptides exposed on the outside membrane of intact mitochondria using succinimidyl ester conjugates. These reactive reagents react with non-protonated aliphatic amine groups, including the amine terminus of proteins and the ε-amino group of lysine residues, which creates a stable carboxamide bond. In another example, when the pharmaceutic agent, e.g., MitoTracker^{®} Orange CMTMRos (Invitrogen, Carlsbad, CA, now Thermo-Fisher Scientific, Cambridge, MA), are mixed with functional mitochondria, they are oxidized and then react with thiols on proteins and peptides on mitochondria to form conjugates.

There are numerous reactive chemical moieties available for attaching therapeutic, diagnostic, and/or imaging agents to the surface of mitochondria (e.g. carboxylic acid, amine functionalized, etc.).

Agents can be attached via protein bonding, amine bonding or other attachment methods either to the outer or inner mitochondrial membrane. Alternatively, or in addition, an agent can be attached to the mitochondria membrane through hydrophobic interaction, Van der Waals interaction, and/or electrostatic interaction.

In many instances, therapeutic agents, diagnostic agents and imaging agents may simply be mixed with isolated mitochondria, and incubated in a buffer (e.g., respiration buffer) for a sufficient period of time (e.g., a few minutes, 5 minutes, 10 minutes, or 1 hour) at favorable conditions (e.g., from 0 °C to 26 °C, from 0 °C to 4 °C, or about 0 °C, 4°C, 26 °C, pH 7.2~8.0).

Exemplary methods of preparing combined mitochondrial agents are described in McCully et al, Injection of isolated mitochondria during early reperfusion for cardioprotection, Am J Physiol 296, H94-H105. PMC2637784 (2009); and Masuzawa et al, Transplantation of autologously derived mitochondria protects the heart from ischemia-reperfusion injury, Am J Physiol 304, H966-982. PMC3625892 (2013).

### Methods of Preparing Compositions Comprising Mitochondria and/or Combined Mitochondrial Agents

Isolated mitochondria and combined mitochondrial agents can be mixed with a pharmaceutically acceptable carrier to make a pharmaceutic composition. A pharmaceutically acceptable carrier includes any compound or composition useful in facilitating storage, stability, administration, cell targeting and/or delivery of the mitochondria and/or combined mitochondrial agent, including, without limitation, suitable vehicles, diluents, solvents, excipients, pH modifiers, salts, colorants, rheology modifiers, lubricants, coatings, fillers, antifoaming agents, polymers, hydrogels, surfactants, emulsifiers, adjuvants, preservatives, phospholipids, fatty acids, mono-, di- and tri-glycerides and derivatives thereof, waxes, oils and water. In some embodiments, isolated mitochondria and/or the combined mitochondrial agents are suspended in water, saline, buffer, respiration buffer, or sterile mitochondria buffer for delivery *in vivo.* Pharmaceutically acceptable salts, buffers or buffer systems, including, without limitation, saline, phosphate buffer, phosphate buffered saline (PBS) or respiration buffer can be included in a composition described herein. Vehicles having the ability to facilitate delivery to a cell *in vivo,* such as liposomes, may be utilized to facilitate delivery of the combined mitochondrial agents to the target cells.

Methods of making compositions, e.g., liquid, semi-solid, and solid compositions (e.g., liquids, creams, lotions, ointments, oils, among others), are well-known in the art. Skilled practitioners will appreciate that such known methods can be modified to add one or more steps to add mitochondria and/or combined mitochondrial agents and form a composition described herein. Skilled practitioners will appreciate that in some instances a composition described herein may include more than one type of combined mitochondrial agent. For example, included are compositions comprising mitochondria wherein essentially each mitochondrion is associated with multiple types of agents. Also included are compositions comprising mitochondria wherein each mitochondrion is paired with only one type of agent but wherein the composition comprises a mixture of mitochondria/agent pairings.

### Treating cardiovascular disease

The heart is a highly energetic organ that requires a continuous supply of oxygen to maintain normal function. Under aerobic conditions, the heart derives its energy primarily from the mitochondria, which constitute 30% of the total myocardial cell volume. Following the onset of ischemia, there is a rapid decline in high-energy phosphate levels with alterations in mitochondrial structure, volume, oxygen consumption, and ATP synthesis.

As disclosed in claim 1, the present disclosure provides a composition comprising isolated mitochondria or a combined mitochondrial agent that comprises isolated mitochondria for use in methods of treating or preventing heart failure. Cardiovascular disease refers to a class of diseases that involve the heart or blood vessels. Cardiovascular diseases include e.g., coronary artery diseases (CAD) such as angina and myocardial infarction (commonly known as a heart attack), stroke, heart failure, hypertensive heart disease, rheumatic heart disease, cardiomyopathy, heart arrhythmia, congenital heart disease, valvular heart disease, carditis, aortic aneurysms, peripheral artery disease, thromboembolic disease, and venous thrombosis etc.

Heart failure, also known as chronic heart failure, refers to a disorder in which the heart is unable to maintain sufficient blood flow to meet the body's needs. Signs and symptoms of heart failure commonly include shortness of breath, excessive tiredness, and leg swelling. A limited ability to exercise is also common among patients with heart failure. As used in this context, to "treat" means to ameliorate at least one symptom of the disorder associated with the disease. Often, the treatment results in an improvement in blood supply, and an improvement of one or more symptoms (e.g., shortness of breath, excessive tiredness, and leg swelling).

Generally, the methods involve administering to a composition (e.g., a composition comprising isolated mitochondria or a composition comprising a combined mitochondrial agent), to a subject who is in need of, or who has been determined to be in need of, such treatment.

In some aspect, as disclosed in claim 5, the present disclosure provides a composition comprising isolated mitochondria or a combined mitochondrial agent that comprises isolated mitochondria for use maintaining right ventricular (RV) contractility, maintaining RV capillary density, preventing RV dilatation, or delaying the onset of RVF.

Described and not claimed *per se* are methods of minimizing heart failure, reducing risk of heart failure, ameliorating at least one symptom of heart failure, preventing or treating cell damage, tissue damage, and/or organ damage associated with heart failure, in a subject at risk of heart failure.

As used herein, the term "at risk of heart failure" refers to an increased risk of heart failure as compared to the risk of heart failure for an average person in the population (e.g., within the same age group). In some embodiments, the risk is about or at least 50%, 60%, 70%, 80%, 90%, or 100% higher than the risk of heart failure for an average person in the population. In some embodiments, the risk is about or at least 2, 3, 4, 5, 6, 7, 8, 9, 10 times higher than the risk of heart failure for an average person in the population. In some embodiment, the age group is at least 40, 50, 60, 70, or 80 years old.

This increased risk of heart failure can be due to various factors, for example, genetic factors (e.g., genetic mutations), environmental factors (e.g., occupation risk, pollution), various diseases, medical procedures (e.g., surgery, organ/tissue transplantation), behaviors (e.g., smoking, inactivity) etc. Once a subject has been identified as having a risk of heart failure, a therapeutically effective amount of composition as described herein can be administered to the subject to reduce the risk of heart failure. The risk can also arise from a potential medical procedure. As used herein, the term "medical procedure" refers to a course of action intended to achieve a result in the delivery of healthcare. The medical procedure can include e.g., diagnostic procedures, therapeutic procedures, and surgical procedures. Some medical procedures include e.g., extracorporeal membrane oxygenation (ECMO), chemotherapy, radiation therapy, tracheal intubation, gene therapy, anesthesia, ablation, amputation, cardiopulmonary resuscitation (CPR), cryosurgery, endoscopic surgery, hemilaminectomy, image-guided surgery, knee cartilage replacement therapy, laminectomy, laparoscopic surgery, lithotomy, lithotriptor, lobotomy, neovaginoplasty, radiosurgery, stereotactic surgery, vaginoplasty, transplantation (e.g., tissue or organ transplantation), xenotransplantation, etc. A healthcare provider can determine whether a medical procedures and a behavior (e.g., smoking) can increase the risk of heart failure. Many risk factors are known in the art, including e.g., hypertension, myocardial infarction, abnormal heart valves, cardiomyopathy, family history of heart disease, and diabetes. In these cases, a therapeutically effective amount of composition as described herein can be administered to the subject before these procedures to minimize the risk.

As disclosed in claim 1, the subject has or is at risk of developing right ventricular hypertrophy (RVH) or right ventricular failure (RVF).

Right ventricular hypertrophy (RVH) is a condition defined by an abnormal enlargement of the cardiac muscle surrounding the right ventricle. RVH usually occurs due to chronic lung disease or structural defects in the heart. One of the most common causes of RVH is pulmonary hypertension (PH). Pulmonary hypertension is characterized as increased blood pressure in the vessels supplying blood to the lungs. Pulmonary hypertension can lead to increased pulmonary artery pressure. The right ventricle tries to compensate for this increased pressure by changing its shape and size. Hypertrophy of individual myocytes results in an increase in right ventricular wall thickness. Common causes of pulmonary hypertension include chronic obstructive pulmonary disease (COPD), pulmonary embolism, and other restrictive lung diseases. RVH often occurs as a result of these disorders. RVH also occurs in response to structural defects in the heart. One common cause is tricuspid insufficiency. Tricuspid insufficiency is a disorder where the tricuspid valve fails to close properly, allowing backward flow of blood. Other structural defects which can lead to RVH include tetralogy of Fallot, ventricular septal defects, pulmonary valve stenosis, and atrial septal defects. RVH is also associated with abdominal obesity, elevated fasting blood glucose, high systolic blood pressure, and fractional shortening of the left ventricular mid-wall. Other risk factors for RVH include smoking, sleep apnea, and strenuous activity.

Thus, in one aspect, the disclosure provides a composition comprising isolated mitochondria or a combined mitochondrial agent that comprises isolated mitochondria for use in methods of reducing the risk of developing right ventricular hypertrophy. The methods involve identifying the subject as being at risk of developing right ventricular hypertrophy, and administering a composition as described herein to the subject. In some embodiments, the methods involve identifying the subject as having e.g., pulmonary hypertension, COPD, pulmonary embolism, restrictive lung diseases, tricuspid insufficiency, tetralogy of Fallot, ventricular septal defects, pulmonary valve stenosis, atrial septal defects, abdominal obesity, elevated fasting blood glucose, high systolic blood pressure, and/or fractional shortening of the left ventricular mid-wall, etc.

Left ventricular hypertrophy (LVH) is thickening of the heart muscle of the left ventricle of the heart. While LVH itself is not a disease, it is usually a marker for disease involving the heart. Disease processes that can cause LVH include any disease that increases the afterload that the heart has to contract against, and some primary diseases of the muscle of the heart. Causes of increased afterload that can cause LVH include aortic stenosis, aortic insufficiency and hypertension. Primary disease of the muscle of the heart that cause LVH are known as hypertrophic cardiomyopathies, which can lead into heart failure. Longstanding mitral insufficiency can also lead to LVH as a compensatory mechanism.

Thus, in one aspect, the disclosure provides a composition comprising isolated mitochondria or a combined mitochondrial agent that comprises isolated mitochondria for use in methods of reducing the risk of developing left ventricular hypertrophy. The methods involve identifying the subject as being at risk of developing left ventricular hypertrophy, and administering a composition as described herein to the subject. In some embodiments, the methods involve identifying the subject as having e.g., aortic stenosis, aortic insufficiency, hypertension, hypertrophic cardiomyopathies, and/or mitral insufficiency etc.

Heart failure (HF), also known as congestive heart failure is when the heart is unable to pump sufficiently to maintain blood flow to meet the body's needs. Signs and symptoms of heart failure commonly include shortness of breath, excessive tiredness, and leg swelling. A limited ability to exercise is also a common feature. Common causes of heart failure include coronary artery disease, including a previous myocardial infarction (heart attack), high blood pressure, atrial fibrillation, valvular heart disease, excess alcohol use, infection, and cardiomyopathy. The left side of the heart receives oxygen-rich blood from the lungs and pumps it forward to the systemic circulation (the rest of the body except for the pulmonary circulation). Failure of the left side of the heart causes blood to back up (be congested) into the lungs, causing respiratory symptoms as well as fatigue due to insufficient supply of oxygenated blood. Right-sided heart failure is often caused by pulmonary heart disease, which is typically caused by difficulties of the pulmonary circulation, such as pulmonary hypertension or pulmonic stenosis.

Thus, in one aspect, the disclosure provides a composition comprising isolated mitochondria or a combined mitochondrial agent that comprises isolated mitochondria for use in methods of treatment or prevention of heart failure. The methods involve identifying the subject as being at risk of developing heart failure, and administering a composition as described herein to the subject. In some embodiments, the subject has RVH, thus has an increased risk of developing heart failure. Described and not claimed *per se* are methods used to treat or reduce the risk of developing pulmonary heart disease or pulmonary disorders (e.g., chronic obstructive pulmonary disease, chronic bronchitis, emphysema, cystic fibrosis, pleural effusion, or bronchiectasis).

Methods of diagnosing cardiovascular disorders are known in the art. One primary method to diagnose cardiovascular disorders is echocardiography, with which the thickness of the muscle of the heart can be measured. For example, the electrocardiogram (ECG) is often used to show signs of increased voltage from the heart in individuals with LVH.

Described and not claimed *per se* are methods of treating ischemic heart and other ischemia-related diseases. Attempts to lessen myocardial tissue necrosis and improve post-ischemic function using pharmacological and/or exogenous substrate interventions, either alone or in combination with procedural techniques, have provided only limited cardioprotection. Despite these interventions, mitochondrial damage and dysfunction continue to represent major problems following myocardial ischemia and remain significant causes of morbidity and mortality. Mitochondrial damage occurs mainly during ischemia rather than during reperfusion, and that preservation of mitochondrial respiratory function enhances contractile recovery and decreases myocardial infarct size.

Described and not claimed *per se* are methods used to treat ischemic heart. For example, an effective amount of isolated mitochondria can be injected into the blood vessel of a subject, for example, the coronary vasculature of the subject. For example, about 1 × 10⁷ of mitochondria can be administered into the coronary vasculature of the subject. The injected mitochondria are internalized by cardiomyocytes after transplantation and provide enhanced oxygen consumption, upregulate chemokines that enhance post-infarct cardiac function, and upregulate the expression of protein pathways that are important in preserving myocardial energetics. In another example, an effective amount of mitochondria can be directly injected to the area at risk (regional ischemic area). The injection can be repeated several times at different sites of the heart.

Reperfusion injury is the tissue damage by blood supply when blood returns to the tissue after a period of ischemia or lack of oxygen. The absence of oxygen and nutrients during the ischemic period results in inflammation and oxidative damage when blood flow is restored. The inflammatory response further leads to the reperfusion injury in the tissue. Therefore, in some instances, a treatment also involves administering immune suppressors to the patient. The immune suppressors can be, e.g., administrated separately, but as a concurrent treatment with the mitochondrial agent. Alternatively, or in addition, the immune suppressors can be linked to mitochondria to form a combined mitochondrial agent, which can be used for the treatment. Particularly useful immune suppressors are bisphosphonates.

The ischemia/reperfusion injury in some other organs is often associated with mitochondrial damage and dysfunction as well. These organs include, but are not limited to, lung, kidney, liver, skeletal muscle, brain, etc. These injuries or diseases include, but are not limited to, ischemic colitis, mesenteric ischemia, brain ischemia, stroke, acute limb ischemia, cyanosis and gangrene. The described method can be also employed to treat ischemia injury in these organs/tissues. For these treatments, the isolated mitochondria and/or combined mitochondrial agent can be directly injected to the organ tissue or injected into the blood vessel which carries the blood to the target organ/tissue or the injured site of the subject.

### Heart surgery

The isolated mitochondria and/or combined mitochondrial agents can be delivered to the heart to decrease stunning and allow for weaning of the heart from a surgical procedure (e.g., cardioplegia), and recovery of the heart without increasing heart rate or oxygen demands in the heart. In some embodiments, the composition comprising isolated mitochondria or the combined mitochondrial agent that comprises isolated mitochondria for use involves direct injection of isolated mitochondria and/or combined mitochondrial agents to the heart. In some methods, isolated mitochondria and/or combined mitochondrial agents are injected into a coronary artery.

### Imaging

Imaging agents can be attached to mitochondria, often by co-incubation of the mitochondria with the imaging agents. Such imaging agents include, but are not limited to, MitoTracker and pHrodo fluorophores from Thermo Fisher Scientific Inc., ¹⁸F-Rhodamine 6G, and iron oxide nanoparticles.

Combined mitochondrial agents that include an imaging agent can be injected into the tissue, e.g., heart tissue, or perfused through the blood vessels. Tissues containing the labeled mitochondria can be examined using imaging techniques, such as positron emission tomopgrahy (PET), microcomputed tomography (µCT), and magnetic resonance imaging (MRI), brightfield microscope, and 3-D super-resolution microscopy, etc. Skilled practitioners will appreciate that other imaging techniques or modalities may be used. They include, but are not limited to, x-rays, scintigraphy, fluorescence and ultrasound.

### Administration

Isolated mitochondria and combined mitochondrial agents can be administered to a patient by injection intravenously, intra-arterially, intraperitoneally, intra-muscularly, and/or through intraosseous infusion. In some embodiments, isolated mitochondria and combined mitochondrial agents, can be delivered by direct injection or by vascular infusion.

Once mitochondria are injected into a tissue, mitochondria will be taken up by cells around the site of injection. Therefore, in some embodiments, the site of injection is the target site. In some other embodiments, mitochondria are injected to a blood vessel which carries the blood to the target site, for example, an organ, a tissue, or an injured site. While not intending to be bound by any theory, evidence suggests that mitochondria delivered by direct injection are internalized by cells through actin-dependent endocytosis. However, mitochondrial uptake by vascular delivery appears to be more complicated. The rapid and widespread uptake of mitochondria when delivered by vascular infusion would suggest that mechanisms allowing for the rapid passage of mitochondria through the vascular wall are involved. Some studies support the concept that cells can routinely escape from the circulation. It has been shown that certain cardiac and mesenchymal stem cells appear to be actively expelled from the vasculature in a process different from diapedesis (Cheng, K., Shen, D., Xie, Y., Cingolani, E., Malliaras, K., Marbán, E., 2012, Brief report: Mechanism of extravasation of infused stem cells. Stem Cells. 30, 2835-2842.; Allen, T.A., Gracieux, D., Talib, M., Tokarz, D.A., Hensley, M.T., Cores, J., Vandergriff, A., Tang, J., de Andrade, J.B., Dinh, P.U., Yoder, J.A., Cheng, K., 2017. Angiopellosis as an Alternative Mechanism of Cell Extravasation. Stem Cells. 35,170-180). Transmigration of stem cells through the vascular wall requires extensive remodeling of the endothelium. Mitochondria may use a similar remodeling mechanism to pass through the vascular wall. Another possible mechanism for mitochondrial uptake may be diapedesis- like. Some cells routinely escape from the circulation. For example, leukocyte extravasation (i.e. diapedesis) between venous endothelial cells is a well-understood process that involves cell adhesion proteins. Further, it is also possible that infused mitochondria extravasate through the capillary wall through the space between the endothelium cells. After mitochondria cross the endothelium of the blood vessels, mitochondria are taken up by tissue cells through an endosomal actin-dependent internalization process.

Mitochondria or combined mitochondrial agents can be administered to a subject as a singular, one-time treatment, or alternatively, multiple treatments, e.g., a treatment course that continues intermittently or continuously for about 1, 2, 5, 8, 10, 20, 30, 50, or 60 days, one year, indefinitely, or until a physician determines that administration of the mitochondria or combined mitochondrial agent is no longer necessary.

In one method of administration, mitochondria or combined mitochondrial agents are injected into organ tissue, e.g., heart tissue, directly. The injection can in some instances be repeated several times at different sites of the organ. In such a method, a sterile 1-ml insulin syringe with a small needle (e.g., 28-gauge) can be used for the injection and each injection site can receive, e.g., about 1.2 × 10⁶ of mitochondria.

Skilled practitioners will appreciate that the amount of mitochondria and/or combined mitochondrial agents, e.g., compositions comprising mitochondria and/or combined mitochondrial agents, that should be administered to a patient will vary depending upon, e.g., the type of disorder being treated, the route of administration, the duration of the treatment, the size of an area to be treated, and/or the location of the treatment site in the patent, among others. Skilled practitioners will be able to determine dosages to be administered depending on these and other variables. For example, a total of about 1 × 10⁷ of mitochondria can be administered into a blood vessel of a subject, e.g., to treat localized ischemia in the myocardium. As another example, in the case of larger organs or affected areas, greater numbers of mitochondria, e.g., 1 x 10 ¹⁰ to 1 x 10 ¹⁴ mitochondria, can be injected into the blood vessel. Conversely, in the case of small focal lesions, 1 x 10 ³ to 1 x 10 ⁶ mitochondria can be infused into the patient. Therefore, an effective amount of mitochondria or combined mitochondrial agents (or compositions comprising same) is the total amount of mitochondria or combined mitochondrial agents sufficient to bring about a desired therapeutic effect. An effective amount can be, e.g., at least or about 1 x 10² mitochondria or combined mitochondrial agents e.g., from about 1 x 10³ to about 1 x 10¹⁴, about 1 x 10⁴ to about 1 x 10¹³, about 1 x 10⁵ to about 1 x 10¹², about 1 x 10⁶ to about 1 x 10¹¹, about 1 x 10⁷ to about 1 x 10¹⁰, about 1 x 10³ to about 1 x 10⁷, about 1 x 10⁴ to about 1 x 10⁶, about 1 x 10⁷ to about 1 x 10¹⁴, or about 1 x 10⁸ to about 1 x 10¹³, about 1 x 10⁹ to about 1 x 10¹², about 1 x 10⁵ to about 1 x 10⁸ or at least or about 1 x 10³, 1 x 10 ⁴, 1 x 10⁵, 1 × 10⁶,1 x 10 ⁷, 1 x 10 ⁸, 1 x 10 ⁹, 1 x 10 ¹⁰, 1 x 10 ¹¹, 1 x 10¹², 1 x 10¹³, or at least or about 1 x 10¹⁴, or e.g., an amount more than 1 x 10 ¹⁴. As used herein, the term "total amount" in the context of administration to a patient can refer to the total amount of mitochondria or combined mitochondrial agents in a single administration (e.g., one injection, one dose administered in an infusion) or in multiple administrations (e.g., multiple injections), depending on the dosing regimen being performed.

Isolated mitochondria and/or combined mitochondrial agents can be administered to a subject every 12-24 hours by various routes, e.g., direct injection, vascular delivery. In some embodiments, isolated mitochondria or combined mitochondrial agents can be administered to a subject every 5-10 minutes (e.g., every 5 minutes, every 10 minutes) by various routes, e.g., direct injection, vascular infusion.

To treat cardiovascular diseases or lung diseases, the isolated mitochondria and/or combined mitochondrial agents can be administered into various blood vessels, including e.g., the aorta, vena cava (e.g., superior or inferior vena cava), coronary veins, circumflex artery, left coronary artery, left anterior descending artery, pulmonary veins, right coronary artery, pulmonary vein, or pulmonary artery.

The isolated mitochondria and/or combined mitochondrial agents can be administered to a subject at least or about 1, 2, 3, 4, 5, 6, 7, 8, 9, 10, 20, or 30 days or at least or about 1, 2, 3, 4, 5, 6, 7, 8, 9, 10, 11, 12 months or at least or about 1, 2, 3, 4, or 5 years before the onset of right ventricular hypertrophy (RVH), left ventricular hypertrophy (LVH), right ventricular failure (RVF), or left ventricular failure (LVF). The isolated mitochondria and/or combined mitochondrial agents can be administered to a subject within 1, 2, 3, 4, 5, 6, 7, 8, 9, 10, 20, or 30 days or within 1, 2, 3, 4, 5, 6, 7, 8, 9, 10, 11, 12, 24 months or within about 1, 2, 3, 4, or 5 years after the subject has been identified to be at risk of developing a cardiovascular disease that may lead to heart failure (e.g. obesity, right ventricular hypertrophy, and the like).

The isolated mitochondria and/or combined mitochondrial agents can be administered to a subject within 1, 2, 3, 4, 5, 6, 7, 8, 9, 10, 20, or 30 days or within 1, 2, 3, 4, 5, 6, 7, 8, 9, 10, 11, 12, 24 months or within about 1, 2, 3, 4, or 5 years after the subject has been identified to have a cardiovascular disorder or some other disorders (e.g., obesity, right ventricular hypertrophy, and the like) that may lead to heart failure.

The isolated mitochondria and/or combined mitochondrial agents can be administered to a subject at least or about 1, 2, 3, 4, 5, 6, 7, 8, 9, 10, 20, or 30 days or at least or about 1, 2, 3, 4, 5, 6, 7, 8, 9, 10, 11, 12 months or at least or about 1, 2, 3, 4, or 5 years after the onset and/or diagnosis of right ventricular hypertrophy (RVH), left ventricular hypertrophy (LVH), right ventricular failure (RVF), or left ventricular failure (LVF).

In some embodiments, isolated mitochondria or combined mitochondrial agents can be directly injected into tissues or organs by Gauge 24, 25, 26, 27, 28, 29, 30, 31, 32, 33, and 34 needles. In some other cases, isolated mitochondria, or combined mitochondrial agents can be delivered to a target site by a catheter.

In some instances, the mitochondria are freshly isolated and viable. The mitochondria or combined mitochondrial agents can be administered to a subject within about 5 minutes, about 10 minutes, about 20 minutes, about 30 minutes, about 40 minutes, about 50 minutes, about 60 minutes, about 70 minutes, about 80 minutes, about 90 minutes, about 100 minutes, about 110 minutes, about 120 minutes after the mitochondria are isolated. In some instances, the mitochondria or combined mitochondrial agents are administered to a subject within about 5 minutes, about 10 minutes, about 20 minutes, about 30 minutes, about 40 minutes, about 50 minutes, about 60 minutes, about 70 minutes, about 80 minutes, about 90 minutes, about 100 minutes, about 110 minutes, about 120 minutes after starting the mitochondria isolating process. Mitochondria and/or combined mitochondrial agents may in some instances be stored for a short period of time (e.g., about or at least 10 minutes, about or at least 20 minutes, about or at least 30 minutes, about or at least 40 minutes, about or at least 50 minutes, about or at least 60 minutes, about or at least 1 hour, about or at least 2 hours, about or at least 3 hours, about or at least 4 hours, or about or at least 24 hours) before use.

The mitochondria for the treatment can be isolated from cells or tissues of an autogenous source, an allogeneic source, and a xenogeneic source. In some instances, mitochondria are collected from cultured cells or tissues of a subject, and these mitochondria are administered back to the same subject. In some other cases, mitochondria are collected from cultured cells or tissues of a second subject, and these mitochondria are administered to a first subject. In some cases, mitochondria are collected from cultured cells or tissues from a different species (e.g., mice, swine, yeast).

### EXAMPLES

The invention is further described in the following examples, which do not limit the scope of the invention described in the claims.

### Example 1: Exemplary Methods of Isolating Mitochondria from Tissue Samples or Cultured Cells

### Preparation

The following solutions can be prepared to isolate intact, viable, respiration-competent mitochondria. To successfully isolate mitochondria using the present methods, solutions and tissue samples are kept on ice to preserve mitochondrial viability. Even when maintained on ice, isolated mitochondria will exhibit a decrease in functional activity over time (Olson et al., J Biol Chem 242:325-332, 1967). These solutions are pre-prepared if possible.
1 M K-HEPES Stock Solution (adjust pH to 7.2 with KOH).
0.5 M K-EGTA Stock Solution (adjust pH to 8.0 with KOH).
1 M KH₂PO₄ Stock Solution.
1 M MgCl₂ Stock Solution.

Homogenizing Buffer (pH 7.2): 300 mM sucrose, 10 mM K-HEPES, and 1 mM K-EGTA. The buffer can be stored at 4°C.

Respiration Buffer: 250 mM sucrose, 2 mM KH₂PO₄, 10 mM MgCl₂, 20 mM K-HEPES Buffer (pH 7.2), and 0.5 mM K-EGTA (pH 8.0). The buffer can be stored at 4°C.

10X PBS Stock Solution: 80 g of NaCl, 2 g of KCl, 14.4 g of Na₂HPO₄, and 2.4 g of KH₂PO₄ are dissolved in 1 L double distilled H₂O (pH 7.4).

1X PBS is prepared by pipetting 100 mL 10X PBS into 1 L double distilled H₂O.

Subtilisin A Stock is prepared by weighing out 4 mg of Subtilisin A into a 1.5 mL microfuge tube. The stock can be stored at -20°C until use.

BSA Stock is prepared by weighing out 20 mg of BSA into a 1.5 mL microfuge tube. The stock can be stored at -20°C until use.

### Isolate mitochondria from tissue

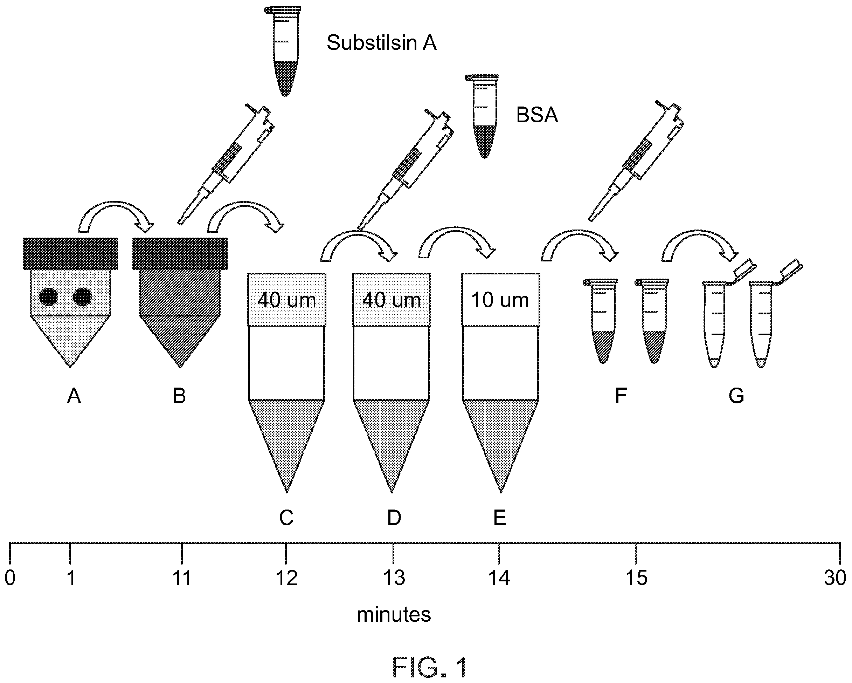

A figure outlining the procedural steps in the isolation of mitochondria using tissue dissociation and differential filtration is shown in **FIG. 1****.** Two, 6 mm biopsy sample punches are transferred to 5 mL of Homogenizing Buffer in a dissociation C tube and the samples are homogenized using the tissue dissociator's 1-minute homogenization program **(A).** Subtilisin A stock solution (250 µL) are added to the homogenate in the dissociation C tube and incubated on ice for 10 minutes **(B).** The homogenated are centrifuged at 750 x G for 4 minutes (as an optional step). The homogenate is filtered through a pre-wetted 40 µm mesh filter in a 50 mL conical centrifuge tube on ice and then 250 µL of BSA stock solution is added to the filtrate **(C).** The filtrate is re-filtered through a new pre-wetted 40 µm mesh filter in a 50 mL conical centrifuge on ice **(D).** The filtrate is re-filtered through a new pre wetted 10 µm mesh filter in a 50 mL conical centrifuge tube on ice **(E).** The filtrate is re-filtered through a new pre wetted 6 µm mesh filter in a 50 mL conical centrifuge tube on ice. The resulting filtrate can be used immediately or can be concentrated by centrifugation. In the case of concentration, the filtrate is transferred to 1.5 mL microfuge tubes and centrifuged at 9000 x g for 10 minutes at 4°C **(F).** The supernatant is removed, and pellets containing mitochondria are re-suspended, and combined in 1 mL of Respiration Buffer **(G).**

Immediately prior to isolation, Subtilisin A is dissolved in 1 mL of Homogenizing Buffer. Immediately prior to isolation, BSA is dissolved in 1 mL of Homogenizing Buffer. Two fresh tissue samples are collected using a 6 mm biopsy sample punch and stored in 1X PBS in a 50 mL conical centrifuge tube on ice. The two 6 mm punches of tissue are transferred to a dissociation C tube containing 5 mL of ice cold Homogenizing Buffer. The tissue is homogenized by fitting the dissociation C tube on the tissue dissociator and selecting the pre-set mitochondrial isolation cycle (60 second homogenization).

The dissociation C tube is removed to an ice-bucket. Subtilisin A Stock Solution (250 µL) is added to the homogenate, mixed by inversion, and the homogenate is incubated on ice for ten minutes. A 40 µm mesh filter is placed onto a 50 mL conical centrifuge tube on ice and the filter is pre-wet with Homogenizing Buffer, and the homogenate is filtered into the 50 mL conical centrifuge tube on ice.

Freshly prepared BSA Stock Solution (250 µL) is added to the filtrate and mixed by inversion. (This step is omitted if mitochondrial protein determination is required.) A 40 µm mesh filter is placed onto a 50 mL conical centrifuge tube on ice and the filter is pre-wet with Homogenizing Buffer, and the homogenate is filtered into the 50 mL conical centrifuge tube on ice. A 10 µm filter is placed onto the 50 mL conical centrifuge tube on ice, and the filter is pre-wetted with Homogenizing Buffer, and the homogenate is filtered into the 50 mL conical centrifuge tube on ice. The filtrate is transferred to two pre-chilled 1.5 mL microfuge tubes and centrifuge at 9000 x g for 10 minutes at 4°C. The supernatant is removed, and the pellets are re-suspended and combined in 1 mL of ice-cold Respiration Buffer.

Mitochondria isolated from tissues are immediately used for injection or to prepare combined mitochondrial agents.

### Isolate mitochondria from cultured cells

Mitochondria can be isolated from cultured cells. The procedure are essentially the same as the procedure for isolating mitochondria from tissue samples, except that cultured cells, e.g., human fibroblasts, are used rather than biopsy samples.

### Mitochondrial number

Viable mitochondrial number are determined by labeling an aliquot (10 µl) of isolated mitochondria with MitoTracker Orange CMTMRos or MitoTracker Red CMXRos (5 µmol/l; Invitrogen, Carlsbad, CA, now Thermo-Fisher Scientific, Cambridge, MA). Aliquots of labeled mitochondria are spotted onto slides and counted using a spinning disk confocal microscope with a 63× C-apochromat objective (1.2 W Korr/0.17 NA, Zeiss). Mitochondria are counterstained with the mitochondria-specific dye MitoFluor Green or MitoTracker Deep Red FM (Invitrogen, Carlsbad, CA, now Thermo-Fisher Scientific, Cambridge, MA). Appropriate wavelengths are chosen for measurement of autofluorescence and background fluorescence with use of unstained cells and tissue. Briefly, 1 µl of labeled mitochondria is placed on a microscope slide and covered. Mitochondrial number is determined at low (×10) magnification covering the full specimen area using MetaMorph Imaging Analysis software.

### Example 2: Exemplary Methods of Preparing Combined Mitochondrial Agents

### Combine mitochondria with ¹⁸F-Rhodamine 6G by electric potential

¹⁸F-Rhodamine 6G (40-100 µCi in a volume of 20 µl) are diluted with mitochondrial isolation solution A (Homogenizing Buffer: 300 mM sucrose, 10 mM K-HEPES, and 1 mM K-EGTA, pH 7.2) at 4°C to a volume of 1.0 mL and then fully mixed with isolated mitochondria (0.5 ml. containing 1x10⁷-1x 10⁸) in mitochondrial isolation solution A. In the mixture, ¹⁸F-Rhodamine 6G distributes electrophoretically into the mitochondrial matrix in response to the electric potential across the inner mitochondrial membrane, and therefore is sequestered by functioning mitochondria. The mixture is incubated on ice for 10-30 minutes. The mixture is washed 3 times by centrifugation at 9,000 rpm (10,000g) for 10 minutes and the pellet is resuspended each time in mitochondrial isolation solution A. Following the final wash, the pellet is resuspended in Respiration Buffer.

### Combine mitochondria with iron oxide nanoparticles by mitochondrial outer membrane

Iron oxide nanoparticles containing a succinimidyl ester (10 mg) are suspended in respiration buffer at 4°C and then fully mixed with isolated mitochondria (1.0 ml containing 1x10⁷-1x 10⁸). Iron oxide is bound to the mitochondrial amine groups on the mitochondrial outer membrane by a succinimidyl ester amine reaction. The mixture is incubated on ice for 10-30 minutes. The mixture is washed 3 times by centrifugation at 9,000 rpm (10,000g) for 10 minutes and the pellet is resuspended each time in mitochondrial isolation solution A. Following the final wash, the pellet is resuspended in Respiration Buffer.

### Combine mitochondria with two pharmaceutical agents

¹⁸F-Rhodamine 6G (40-100 µCi in a volume of 20 µl) and iron oxide nanoparticles containing a succinimidyl ester (10 mg) are combined and diluted with mitochondrial isolation solution A at 4°C to a volume of 1.0 mL and then fully mixed with isolated mitochondria (0.5 ml. containing 1x10⁷-1x 10⁸) in mitochondrial isolation solution. The mixture is incubated on ice for 10-30 minutes. The mixture is washed 3 times by centrifugation at 9,000 rpm (10,000g) for 10 minutes and the pellet is resuspended each time in mitochondrial isolation solution A. Following the final wash, the pellet is resuspended in Respiration Buffer.

### Combine mitochondria through thiols

MitoTracker^{®} fluorophore (5 µmol/l; Invitrogen, Carlsbad, CA, now Thermo-Fisher Scientific, Cambridge, MA) is mixed with isolated mitochondria (1.0 mL) in respiration buffer. When the probes are mixed with functional mitochondria, they are oxidized and then react with thiols on proteins and peptides on mitochondria to form conjugates. The mixture is incubated on ice for 10 minutes at 4 ⁰C in the dark. The mixture is washed 3 times by centrifugation at 9,000 rpm (10,000g) for 10 minutes and the pellet resuspended each time in mitochondrial isolation solution A. Following the final wash, the pellet is resuspended in Respiration Buffer.

### Example 3: Preventing heart failure in pressure-overload hypertrophy through transplantation of autologous mitochondria

### Objectives:

A key event in the progression of right ventricular hypertrophy (RVH) to failure (RVF) is cardiomyocyte apoptosis due to mitochondrial dysfunction. With transplantation of respiration-competent mitochondria available, the aim of these experiments was to determine whether injection of autologous mitochondria could prevent heart failure.

### Methods:

An RVH/RVF model was created by banding of the pulmonary artery by 50% (gradient=15-20mmHg) in immature piglets (n=6/group). Sham-operated animals served as control (Ctr). Animals were followed for 8 weeks by echocardiography (RV free wall thickness measured in M-Mode, TAPSE). Four weeks after surgery, banded animals were either treated with mitochondria (PAB-mito), isolated from the piglet's own calf muscle, or vehicle (PAB-V) through injection into the free-wall of the RV. At euthanasia, tissue was analyzed histologically to determine cardiomyocyte hypertrophy, fibrosis (H&E, Masson's Trichrome, desmin), and apoptosis by TUNEL. Invasive PV loop measurements (Ved, Dp/Dt max, Pdev) were obtained at baseline and at time of euthanasia.

### Results:

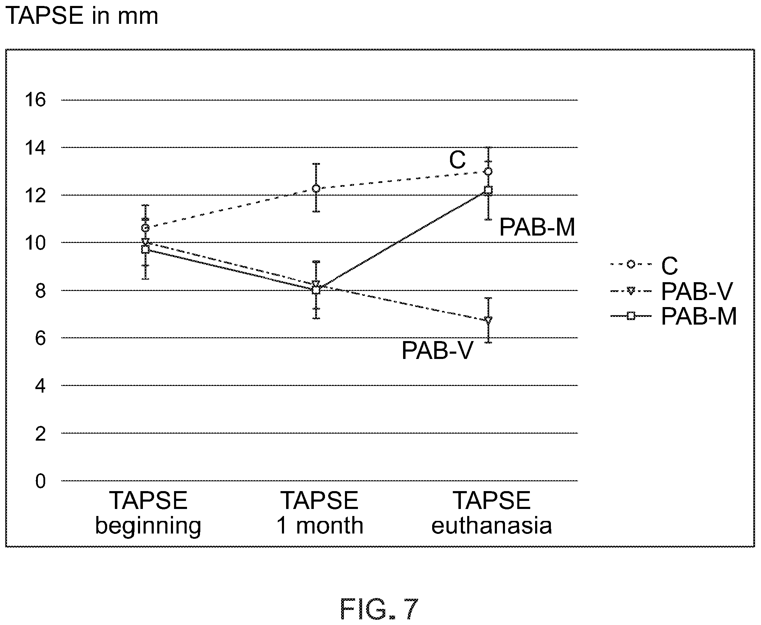

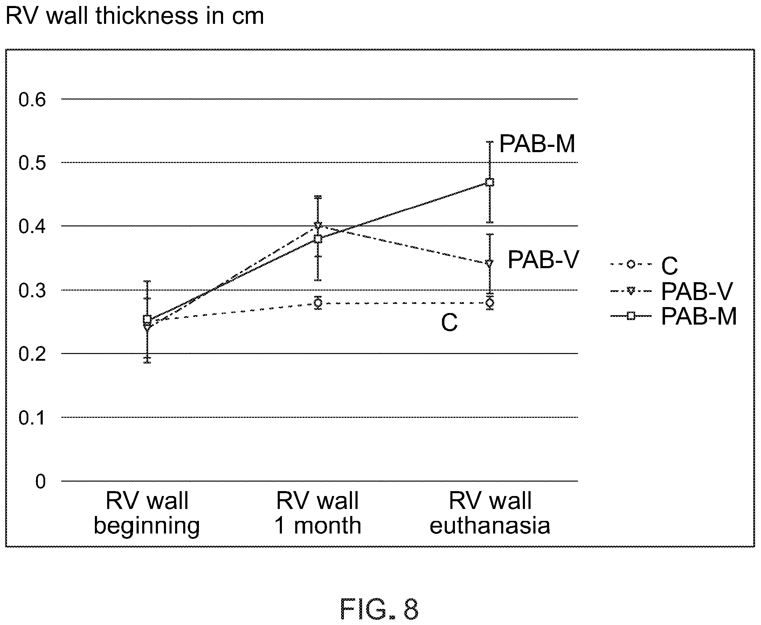

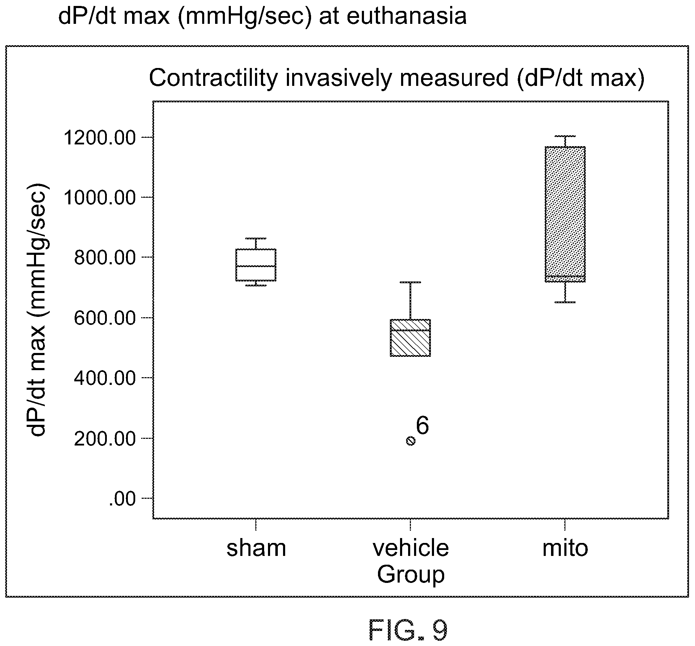

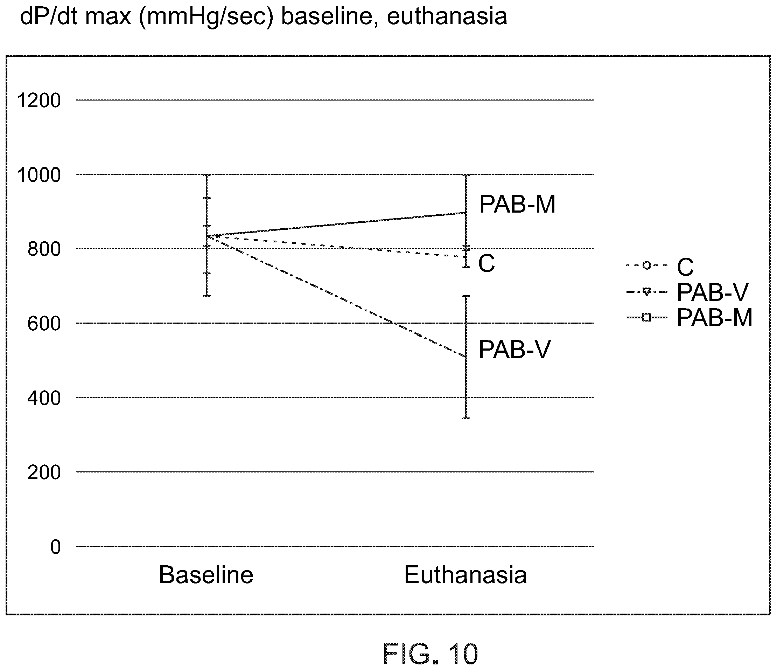

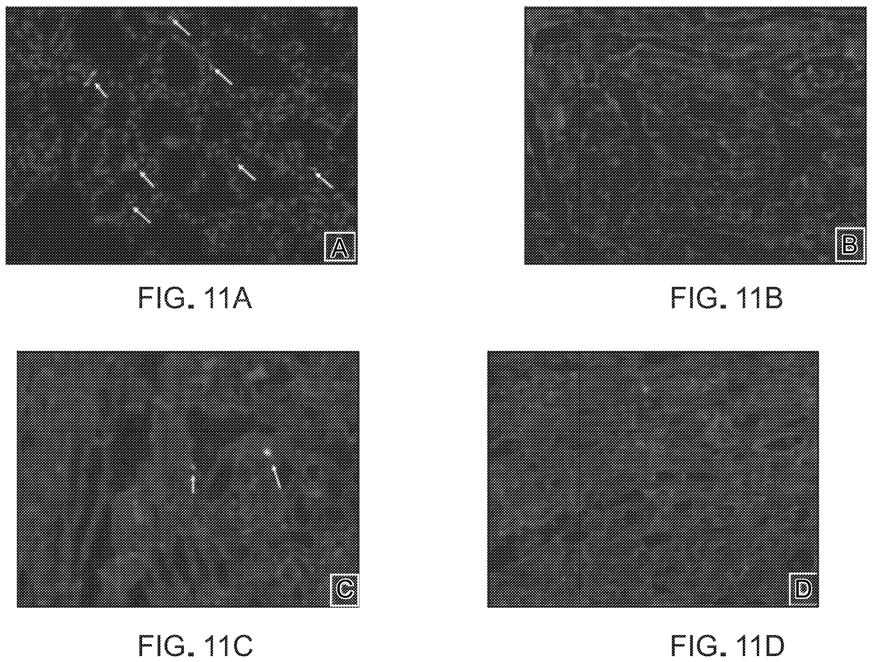

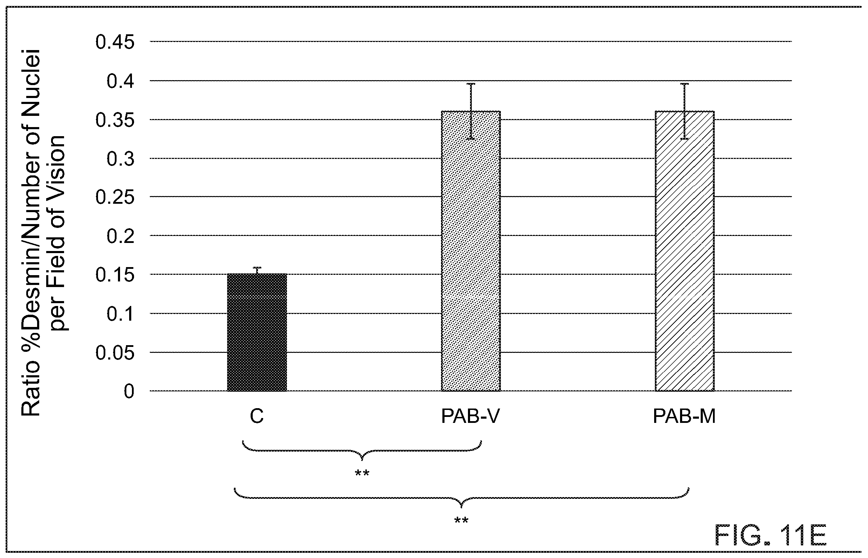

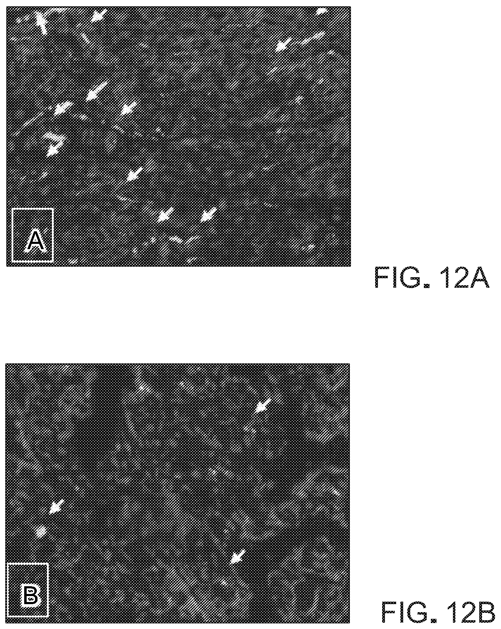

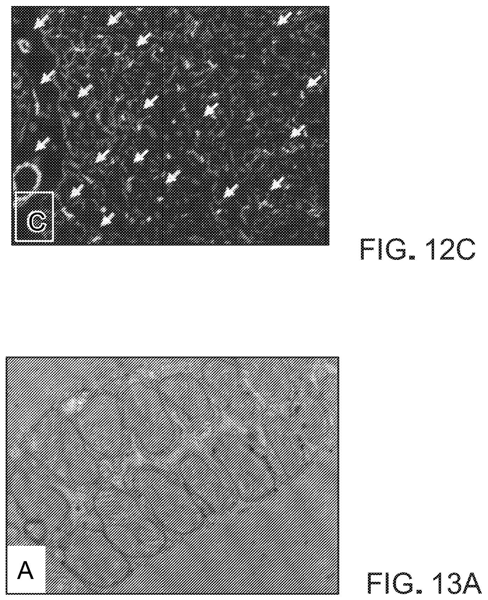

All animals survived until study endpoint. One month after surgery, banded animals showed signs of hypertrophy with a significantly thicker RV free-wall compared to Ctr (0.27±0.03 cm vs. 0.4±0.02 cm; P<0.01; **FIG. 8****).** RV wall thickness further increased until study endpoint in the PAB-mito animals whereas PAB-V hearts were already severely dilated (0.5±0.04 cm vs. 0.28±0.08 cm; P<0.01; **FIG. 8****).** Total heart weight (Ctr: 100.6+11.4g, PAB-V:132.4±31.9g, PAB-mito:141.5±31.4g; P<0.05) and histological hypertrophy calculations (desmin/nuclei ratio: Ctr: 0.17±0.02, PAB-V: 0.45±0.01, PAB-mito: 0.42±0.; P<0.05; **FIGS. 11A-11E****)** corresponded with these findings. There was no apoptotic cardiomyocyte loss in Ctr and PAB-mito hearts but 3±1/total nuclei in the PAB-V hearts **(****FIGS. 12A-12C****).** Dp/Dtmax significantly increased from 831.9±170.5 in all groups at baseline to 1006±178.2 in PAB-mito compared to a decline in PAB-V (501.2±158.9) and remained unchanged in Ctr (843.5±27.6) hearts at time of euthanasia (P<0.05) **(****FIGS. 9-10****).** TAPSE at baseline (10.3±1.7mm) significantly decreased in PAB-V hearts (6.5±0.6mm) compared to a significant improvement in PAB-mito (12.3±1.1mm) hearts (P<0.01) **(****FIG. 7****).**

### Conclusion:

Mitochondrial transplantation maintained hypertrophic adaptation of the RV and preserved contractile function. Addressing the myocardial dysfunction directly by targeting mitochondrial dysfunction can be used to treat patients with pulmonary disease affecting right heart function.

### Example 4: Transplantation of Autogenous Mitochondria for Treatment of Right Heart Failure

Right ventricular hypertrophy (RVH) and failure (RVF) are major causes of cardiac morbidity and mortality. A key event in the progression to RVF is cardiomyocyte apoptosis due to mitochondrial dysfunction. With transplantation of respiration-competent mitochondria available, the aim of this study was to determine whether localized intramyocardial injection of autologous mitochondria can treat heart failure.

In cultured hypertrophied cardiomyocyte beneficial effects of transplanted mitochondria from different sources were determined. An RVH/RVF model through banding of the pulmonary artery in immature piglets with sham-operated controls (n=6/group) was used for treatment with autologous mitochondria (PAB-M), isolated from the piglet's own calf muscle, versus vehicle (PAB-V) injection into the free-wall of the RV. Animals were followed for 8 weeks by echocardiography (free-wall thickness, contractile function) and Dp/Dt max was measured at study endpoint when histological analysis for cardiomyocyte hypertrophy, fibrosis and apoptosis were performed. There was no significant difference in which source of mitochondria was used, neither with internalization nor ATP levels. At 4wks, banded animals showed RVH (C 0.27±0.03cm vs. PAB 0.4±0.02cm wall thickness; P=0.01) which further increased in PAB-M but PAB-V were already severely dilated (0.5±0.04 cm vs. 0.28±0.08 cm; P=0.01). Contractile function at baseline was not different but significantly decreased in PAB-V hearts compared to a significant improvement in PAB-M which was also reflected by Dp/Dtmax at study endpoint. There was negligible apoptotic cardiomyocyte loss and fibrosis in C but significant loss and fibrosis in hypertrophied hearts with highest numbers in PAB-V hearts (C: 1±0.4 versus PAB-V: 13±1.6; p=0.001 and versus PAB-M: 8±1.9; p=0.01. PAB-V versus PAB-M p=0.05). Addressing the myocardial dysfunction directly through mitochondrial transplantation, maintains hypertrophic adaptation of the RV and preserves contractile function.

### Methods and results:

### Animal Model

Immature male Yorkshire piglets (N=18), weighing 5-10kg underwent pulmonary artery banding (PAB) or sham-operation. Piglets were sedated with telazol (4.5mg/kg im), xylazine (2mg/kg im) and atropine (0.04mg/kg im). Following orotracheal intubation, ventilation was started with isoflurane (1-3%) and air. EKG, blood oxygen saturation (maintained at >97%), body temperature, and end-tidal carbon dioxide were monitored. Femoral artery and venous lines were placed. Piglets were placed on their right side, were draped and a left thoracotomy was performed in the fourth intercostal space. Lidocaine (1%, iv) was administered prior to thoracotomy to prevent ventricular fibrillation. The pulmonary artery (PA) was dissected from the ascending aorta and a band was placed while monitoring RV and distal PA pressures through needle puncture. The PA was banded to 50% of its original diameter. A 4F angiographic catheter (Merit Medical Systems, South Jordan, UT) was inserted into the PA and connected to the PowerLab data acquisition system (DAQ, ADInstruments, Series 16/35) to acquire data for baseline pressure calculations. Baseline echocardiography was obtained epicardially before and after placing the PA band. The thoracotomy was closed in three layers and pleural air was evacuated over chest tube. Bupivacaine (0.25%; <0.03mg/kg) was instilled as local analgesic and post-operative systemic analgesia was provided through benamine/glunixin meglumine (1-2mg/kg im) and a fentanyl patch (1-4ug/kg transdermal) for the first 72 hours. Piglets were allowed to recover in an incubator at 37°C and brought back to their pen shortly afterwards. Sham-operation (C, N=6) involved opening and closing the chest with local manipulation at the site of the PA. Following intraoperative echocardiography, progression of RV hypertrophy was determined every other week. During these procedures, the animals were maintained under isoflurane (1-3%) sedation.

Four weeks after PAB, animals were either treated by direct injection of vehicle (PAB-V, N=6) or autologous mitochondria (PAB-M, N=6), into the RV free wall following the same anesthesia protocol as above. Under sterile conditions, a muscle biopsy from the piglets' gastrocnemius muscle was obtained, and mitochondria were isolated as described above. Under direct vision through a subxyphoid approach, either 1ml of buffer (300mM sucrose, 10mM K-HEPES, and 1mM K-EGTA at pH 7.2 and 4°C) containing 10×106/ml autologous mitochondria (PAB-M, N=6) or buffer only (PAB-V, N=6) was injected onto 10 spots into the RV free wall using a 30G needle. Echocardiography was performed prior to injection and directly after mitochondria/vehicle injection. The incision was closed in layers and the animal recovered.

All animals were survived for another 4 weeks (8 weeks after initial PAB) and monitored by echocardiography every other week. At study end point, piglets were anesthetized in the same manner as described above except anesthesia was maintained via face mask rather than intubation. A median sternotomy was performed, and PA (proximally and distally of the PA band) and aortic pressure were measured invasively using a 4F angiographic catheter (Merit Medical Systems, South Jordan, UT). In addition, PA and RV pressure-volume curves were calculated from data obtained using a 7F Scisense Pressure-Volume (PV) loop catheter (Transonic, Ithaca, NY) which was inserted through the RVOT. The PV loop catheter was connected to Powerlab and the signal was automatically calibrated using ADVantage pressure-volume system (ADV500, Transonic, Ithaca, NY). After taking all measurements, Fetal Plus^{®} was administered for euthanasia, and the heart was excised and flushed with phosphate buffered saline (PBS) on ice. RV free wall biopsies were obtained, snap frozen and stored in liquid nitrogen until further usage. Separate RV free wall tissue was embedded in optimal cutting temperature (OCT) compound, snap frozen and stored at -80°C until sectioning was performed. Fresh RV free wall was used wet/dry weight calculation.

### Isolated Cardiomyocyte Cell Culture Model

A cell culture model of neonatal rat cardiomyocytes was used to determine internalization of mitochondria in hypertrophied cardiomyocytes. Furthermore, functional benefits of different sources of mitochondria were tested in this model. We compared pharmacologically-induced hypertrophy samples with non-hypertrophied control samples. All isolated cell experiments were performed in duplicates.

In brief, neonatal rat cardiomyocytes were isolated using a commercially available isolation kit (Worthington) and cultured as previously described in more detail (Choi YH, Stamm C, Hammer PE, et al. Cardiac conduction through engineered tissue. Am J Pathol. 2006;169:72-85). Following two days of culture, the cells were assigned to either control or hypertrophy. To stimulate cardiac hypertrophy *in vitro,* cardiomyocytes were treated with angiotensin II (100nM; Sigma-Aldrich) for 24 hours following serum-starvation for 24 hours.

### Isolation of Mitochondria from Muscle Tissue

To determine whether the source of mitochondria plays a role in the benefits of restoring mitochondrial function, mitochondria from two different skeletal muscle sources, fast-twitch and slow-twitch, were harvested by punch biopsy from gastrocnemius muscle and soleus muscle obtained from the mother rat and were compared to RV cardiac muscle mitochondria. This method yields >99.5% viable mitochondria from a 100mg tissue sample. Muscle tissue was homogenized with a commercial tissue dissociator in homogenizing buffer (300mM sucrose, 10mM K-HEPES, and 1mM K-EGTA at pH 7.2 and 4°C), followed by addition of 250µl buffer solution containing subtilisin A in 1ml of homogenizing buffer. The homogenate was mixed by inversion and incubated on ice for 10min followed by differential filtration. The filtrate was transferred to two pre-chilled microfuge tubes and centrifuges at 9,000xg for 10min at 4°C. The supernatant was removed and combined pellets were re-suspended in 1ml ice-cold respiration buffer (250mmol/l sucrose, 2mmol/l KH2PO4, 10mmol/l MgCl₂, 20mmol/l K+-HEPES buffer, pH 7.2, 0.5mmol/l K+-EGTA, pH 8.0, 5mmol/l glutamate, 5mmol/l malate, 8mmol/l succinate, 1mmol/l ADP). The number of mitochondria was counted with a Coulter counter (Beckman Coulter Life Sciences, Indianapolis, IN).

### Determination of Internalization of Transplanted Mitochondria

Mitochondria were pre-labeled with pHrodo red particle label (ThermoFisher, Waltham, MA) for 10min at 4°C and then washed 4 times in respiration buffer. PHrodo fluorescence provides a positive indication of internalization since it only fluorescence following uptake by viable mitochondria. The labeled mitochondria were re-suspended in fresh respiration buffer and the last wash supernatant was saved. This wash was used to determine non-specific labeling by incubating control cells with this supernatant (data not shown). The labeled mitochondria (1x100/well) were co-incubated with cardiomyocytes (~50,000/well). After 24 hours, the media was removed and cells were washed 4 times with 1x PBS and 200µl of fresh media was added to each well. For staining, cells were permeabilized in 0.1% Triton X-100 in PBS for 3 minutes and incubated with primary antibodies diluted 1:1000 in 10% fetal bovine serum (FBS) in PBS for 1 h. As primary antibodies desmin for cardiomyocytes was used with a species-appropriate Alexa Fluor 488-conjugated secondary antibody (ThermoFisher, Waltham, MA). Nuclei were simultaneously stained using 4',6-diamidino-2-phenylindole (DAPI) (ThermoFisher). Detection of internalization was based on red fluorescent mitochondria into desmin stained cardiomyocytes in green. Internalization was assessed with a Zeiss fluorescent microscope.

### Determination of Mitochondrial Function

ATP content was determined using the ATPlite Luminescence ATP Detection Assay System (Perkin Elmer, Waltham, MA). A separate set of cells was used for these experiments since fluorescent dyes labelling mitochondria might interfere with mitochondrial function.

### Echocardiographic Measurements

Echocardiographic measurements were obtained at baseline, prior to treatment (4 weeks post banding) and at study endpoint (8 weeks post banding). All studies were performed using a Philips iE33 device (Philips Healthcare, Amsterdam, Netherlands) equipped with a S8-3 transducer and all cycles were saved with simultaneous ECG recording. RV function through fractional area change (FAC) and tricuspid annular plane systolic excursion (TAPSE) were assessed from 4-chamber views. RV free wall thickness was measured on M-mode recordings obtained at end-diastole from a parasternal long axis (PLAX) and parasternal short axis (PSA) view.

### Invasive Pressure-Volume (PV) Measurements

PA and RV pressure-volume curves were calculated from data obtained using a 7F Scisense Pressure-Volume (PV) loop catheter (Transonic Systems Inc, Ithaca, NY) which was inserted through the right appendage and secured with a 4-0 Prolene suture (Ethicon Inc., Somerville, NJ). The PV loop catheter was connected and automatically calibrated using ADVantage pressure-volume system (ADV500, Transonic, Ithaca, NY). Measurements were obtained at baseline and at time of euthanasia. Data were acquired using a Powerlab data acquisition system (DAQ, ADInstruments, Series 16/35) and analyzed with the provided software LabChart 7 Acquisition Software (AD Instruments, Sidney, Australia). RV peak developed pressure (Pdev, mmHg), RV end diastolic pressure (Ped, mmHg) and maximal change of RV pressure over time (dp/dt max, mmHg/s) were measured. Maximum volumes (Vmax) and end-diastolic volumes (Ved) were calculated using the PV loop catheter.

### Histological Analysis of RV Tissue

RV tissue was embedded in optimal cutting temperature (OCT) compound, snap frozen and stored at -80°C until further usage. Sections were obtained and frozen slides were stored at -80°C until used for staining. All slides were visualized using a Zeiss Observer.Z1 fluorescent microscope with a Nikon 20x objective (NA=20x/0.45). Ten randomly selected fields from each slide were photographed with a Leica digital color camera and analyzed ImageJ (version 2.0.0-rc-43, obtained from the National Institute of Health, Bethesda, MD).

### Determination of Cardiomyocyte Hypertrophy

In addition to echocardiographic measurements, RV hypertrophy was assessed using immunofluorescence staining for desmin to visualize cardiomyocytes (1:50, Santa Cruz Biotechnology Inc., Dallas, TX) and DAPI (1: 1000, Dako, Carpinteria, CA) to determine the number of nuclei per field of vision. Using ImageJ, the ratio of desmin to number of nuclei per field of vision was calculated.

### Determination of Cardiomyocyte Apoptosis

Without being bound by theory, cardiomyocyte apoptosis has been shown to be mainly a result of mitochondrial dysfunction, and thus, we determined cardiomyocyte apoptosis by TUNEL staining using the ApopTag Plus Fluorescein In Situ Apoptosis Detection Kit (MilliporeSigma, Burlington, MA). Cardiomyocytes were counterstained using desmin (1:50, Santa Cruz Biotechnology Inc., Dellas, TX) and nuclei with DAPI (1: 1000, Dako, Carpinteria, CA). TUNEL-positive nuclei were counted manually. The total number of nuclei per field of vision was determined using ImageJ. Data were expressed as ratio of apoptotic nuclei per 1000 cardiomyocyte nuclei.

### Determination of Myocardial Fibrosis

A separate set of tissue sections was stained with Masson's trichrome, which results in fibrotic (collagen-enriched) areas appearing blue, whereas the myocardium appears red. We measured the blue and red areas (fibrosis versus myocardium) and the ratio serves as an estimate of fibrosis. Slides were visualized with a Nikon 10x objective. Ten randomly selected fields of vision per tissue sample were obtained and analyzed with ImageJ. Results are expressed as the ratio of the blue to red areas.

### Statistical Analysis

All results are reported as mean ± standard error of the mean (SEM). After confirmation of normal distribution of data, ANOVA for multiple group comparisons and Bonferroni's post-hoc analyses were performed to obtain calculations of statistical significance (SPSS 23, IBM Corporation, Armonk, NY). Probability values of ≤ 0.05 were regarded as statistically significant.

### Cardiomyocyte Cell Culture Model

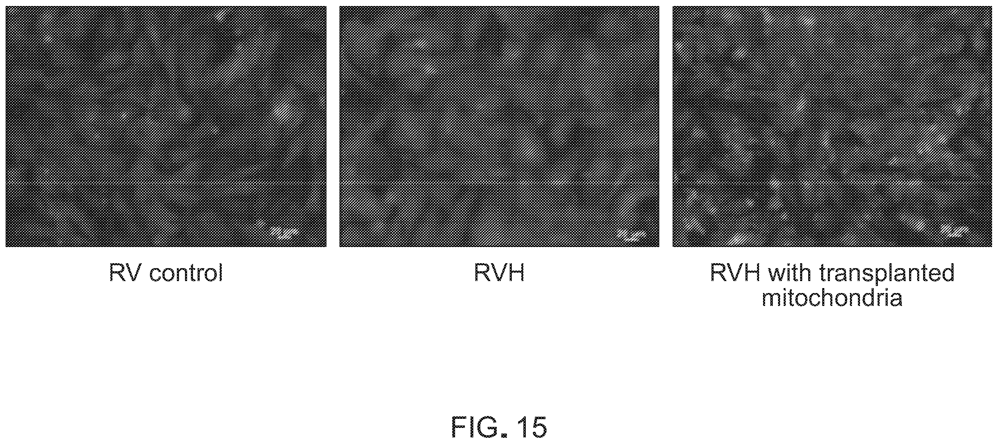

Cardiomyocyte size was determined as an indicator for hypertrophy (H) following exposure to angiotensin II. Following treatment, cardiomyocyte size had significantly increased compared to control cardiomyocytes expressed as ratio per number of nuclei per field of vision (C: 2.4±0.2 versus H: 4.2±0.5; p=0.01). Mitochondria internalized into hypertrophied RV cardiomyocytes in the same magnitude as into control cardiomyocytes **(****FIG. 15****).**

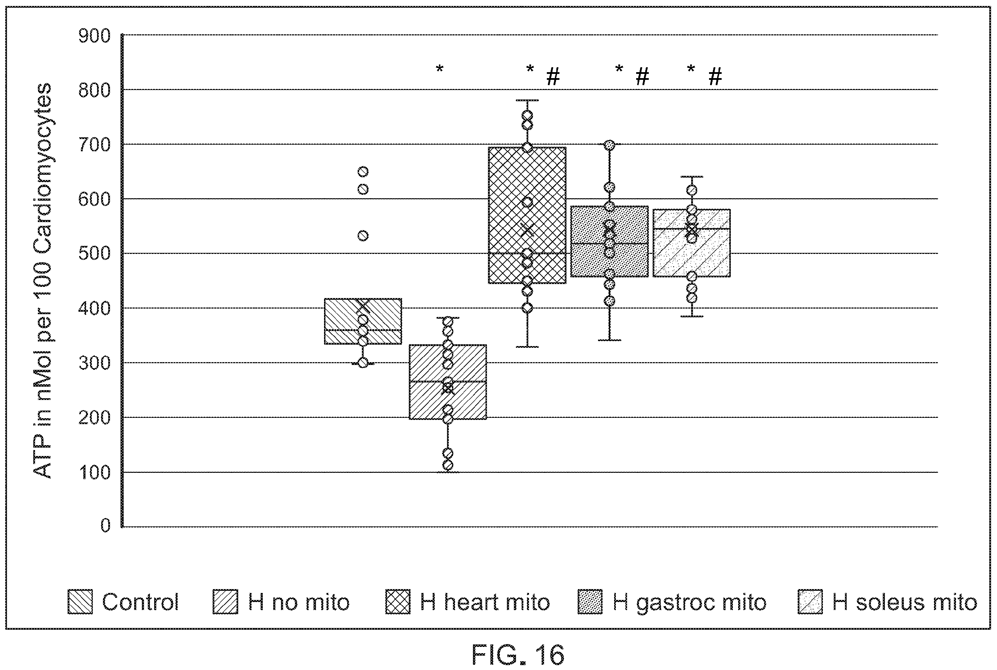

ATP levels expressed per number cardiomyocytes is decreased in hypertrophied cardiomyocytes compared to controls (C: 404±28 versus H-no mito: 256±23; p=0.01) but is normalized to supra-normal levels following transplantation of mitochondria. There is no observed statistical difference which mitochondrial source was used (H-heart mito: 541±36 versus H-gastrocnemius mito: 527±98 versus H-soleus mito: 531±19; n.s.). Skeletal muscle mitochondria responded equally as well as mitochondria isolated from cardiac muscle **(****FIG. 16****).**

### Animal Model

All 18 piglets survived until study endpoint. The average gradient measured across the PA band (mmHg, average±SEM) at study endpoint was not significantly different (P=0.8) between the groups PAB-V (12.1±1.6) and PAB-M (9.7±1.9). Body weight (in kg) did not differ between the three groups neither prior to any intervention (C:12.2±1.5, PAB-V: 11.4±1.5, PAB-M:11.9±0.9; P=0.9) nor at study endpoint (C:17.3±3.1, PAB-V: 16.6±1.2, PAB-M:17.8±0.9; P=0.4). Whole heart weight (in gram) at study end point, however, was significant higher in the PA-banded animals compared to sham-operated controls (C: 100.6+4.7 versus PAB-V: 132.4±13 and PAB-M: 141.6+12.8; P<0.05). RV wet weight/dry weight ratios (wet-dry weight/dry weight) did not differ significantly between three groups (C:4.1±0.05, PAB-V:5.3±0.09, PAB-M:4.9±0.3; P=0.5).

### Histological Analysis

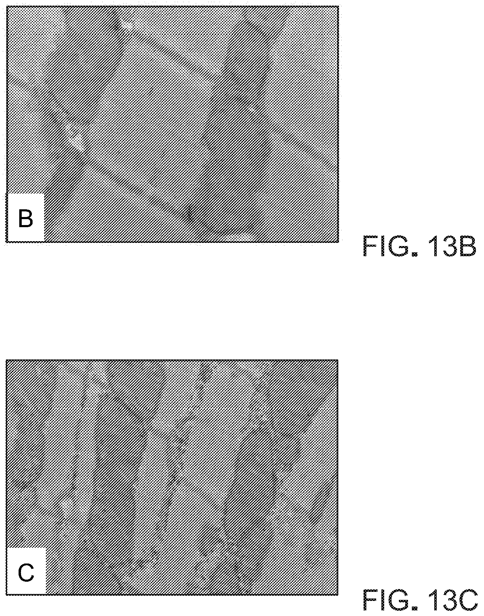

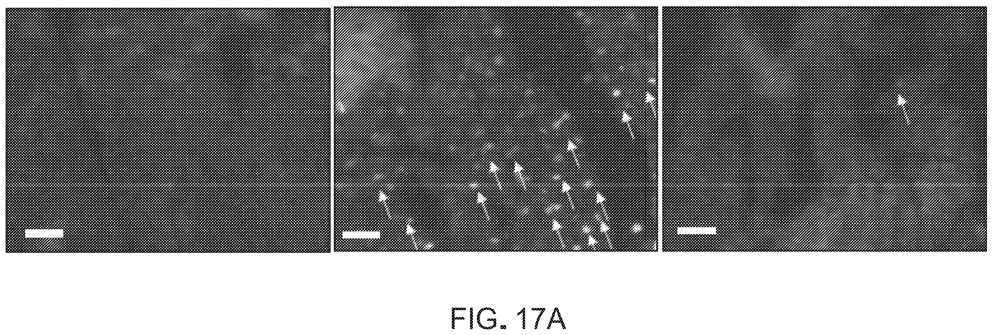

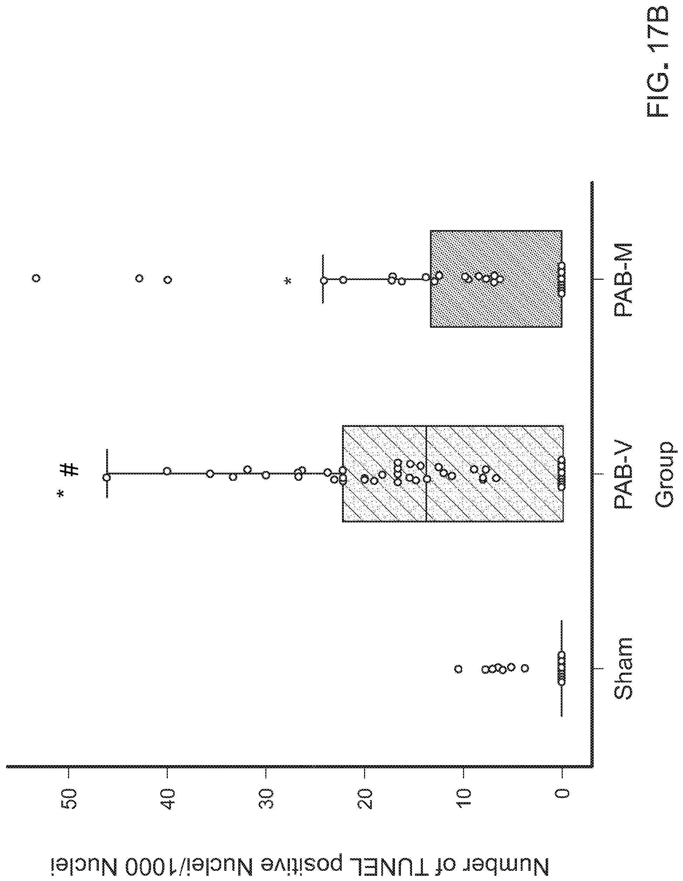

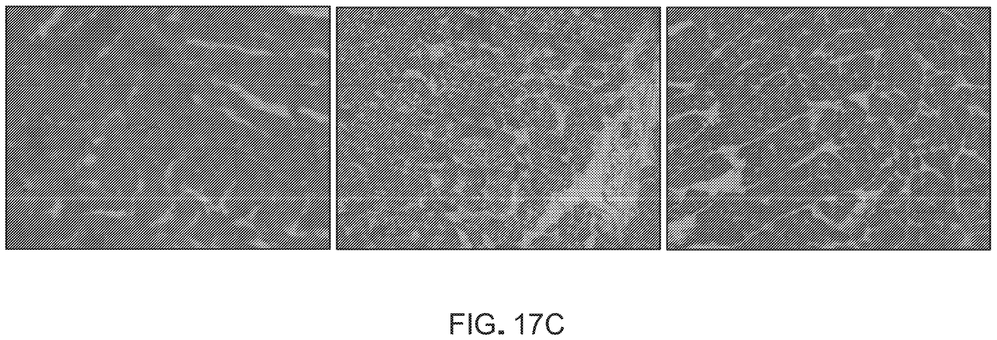

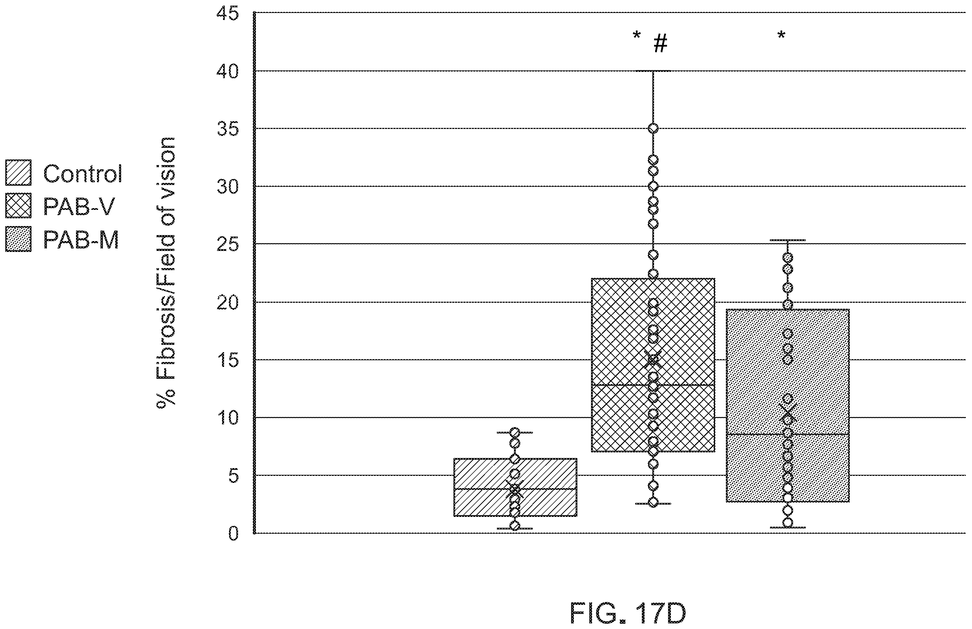

Hypertrophy was assessed histologically by calculating the ratio of myocardial area to number of nuclei per field of vision. Both PAB groups showed significant increase in muscle mass compared to sham-operated controls at study endpoint (C:0.2±0.02 versus PAB-V:0.36±0.02 and PAB-M:0.36±0.3; P=0.001). This finding correlated with the presence of cardiomyocyte apoptosis where PAB-V hearts showed the highest number of apoptosis positive cardiomyocyte nuclei (C: 1±0.4 versus PAB-V: 13±1.7 versus PAB-M: 8±1.9; p≤0.05; **FIGS. 11A-11E** **and** **17A-17B****).** PAB-V mitochondria were also swollen and cristae are reduced **(****FIGS. 13A-13C****).** These findings were also supported by a significant increase in myocardial fibrosis in vehicle treated hypertrophied hearts compared to mitochondria treated hypertrophied hearts (C: 4±0.55 versus PAB-V: 15±1.3 versus PAB-M: 10±1.1; p≤0.05; **FIGS. 17C-17D****).**

### Echocardiographic Measurements

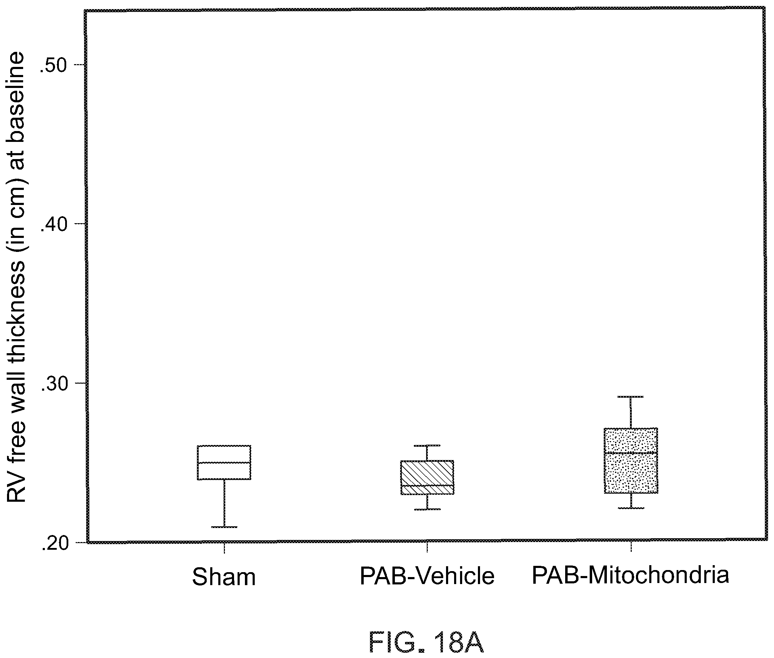

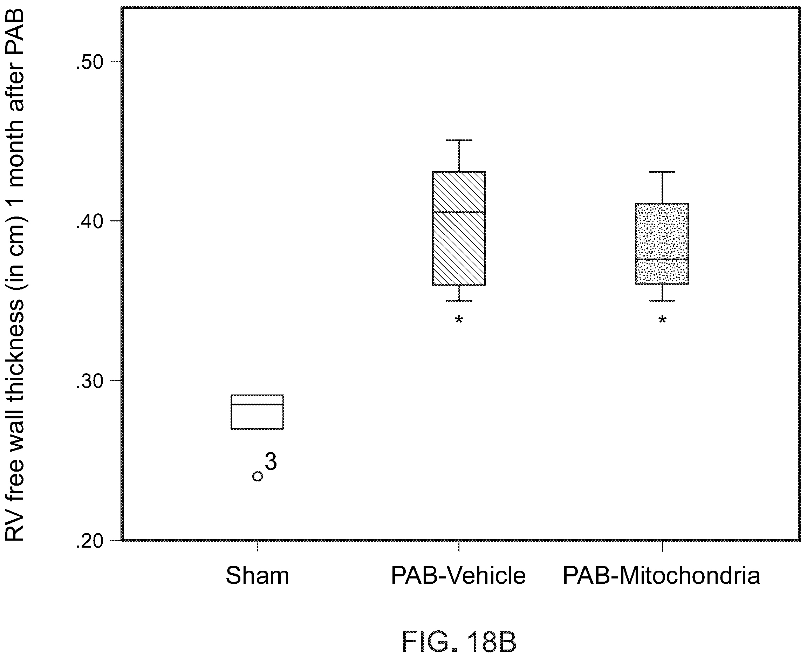

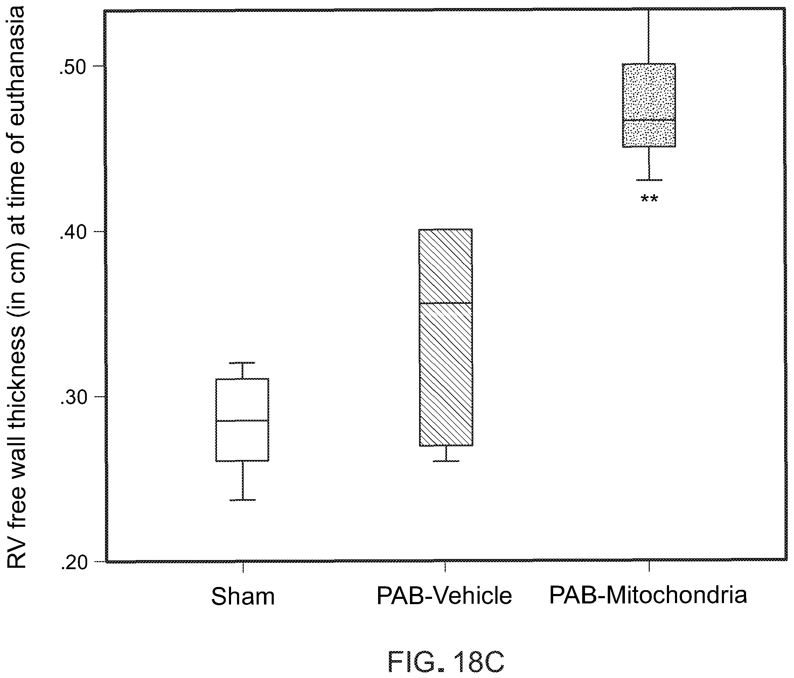

In addition to the histological assessment of cardiomyocyte hypertrophy, right ventricular wall thickness in centimeters was measured on M-mode recordings in end-diastole. Baseline wall thickness was not significantly different between the groups prior to any intervention (C: 0.25±0.01, PAB-V: 0.24±0.01, PAB-M:0.25±0.01; P=0.48) but increased significantly in the banded groups within 4 weeks post banding (C:0.28±0.01 versus PAB-V: 0.4±0.02 and PAB-M:0.38±0.02; P<0.001). At study endpoint, mitochondria treated hearts maintained their wall thickness whereas vehicle treated hearts were back to baseline indicative of dilation (C:0.28±0.01 versus PAB-V:0.34±0.03; P=0.15; versus PAB-M:0.47±0.02; P=0.05). **(****FIGS. 8** **and** **18A****-18C)**

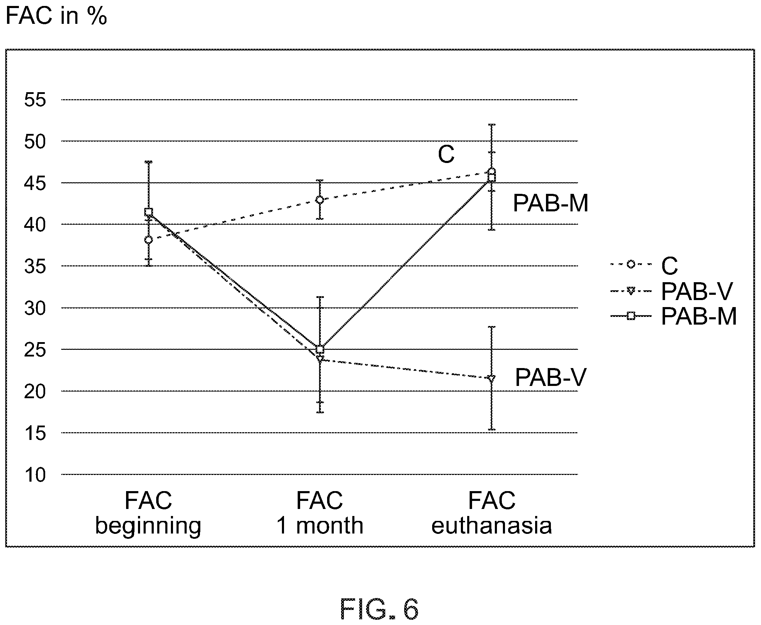

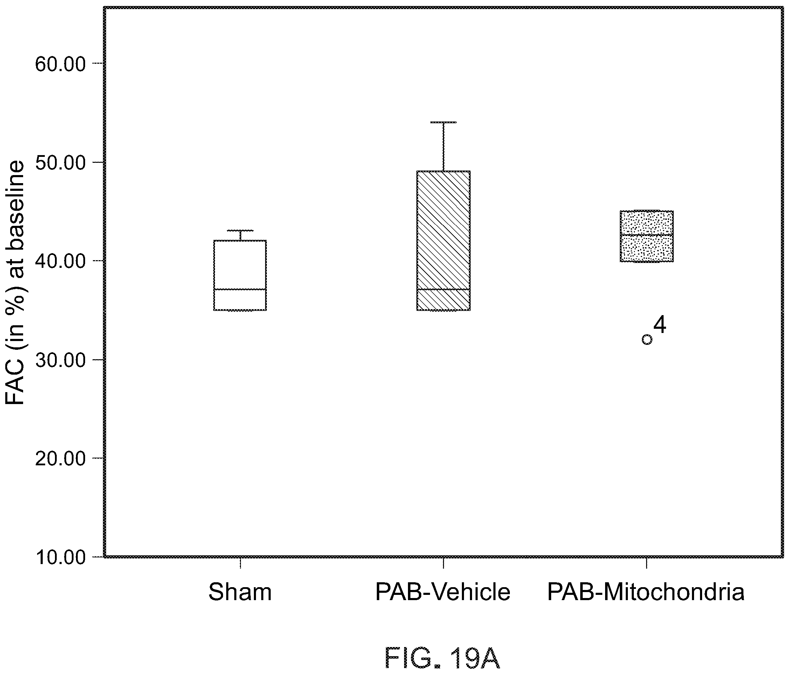

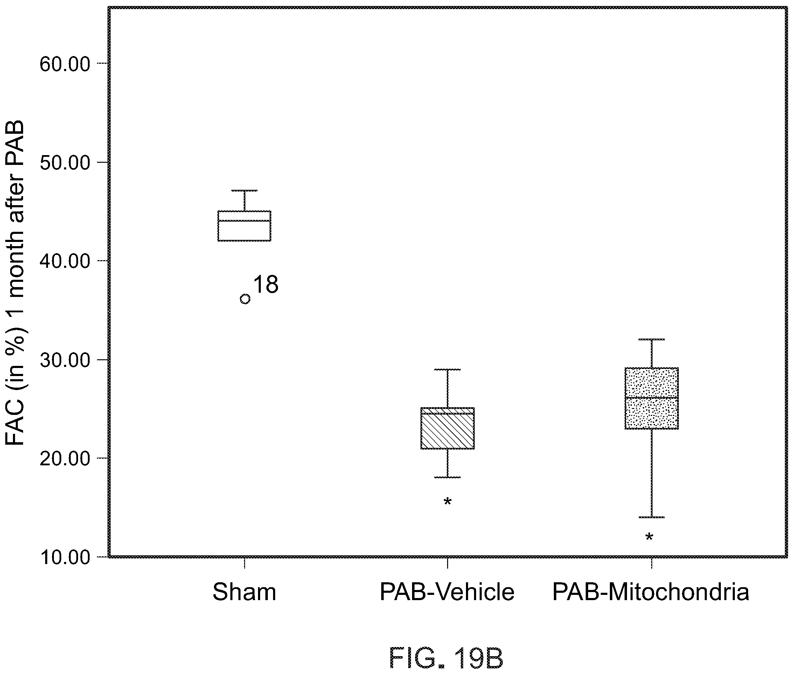

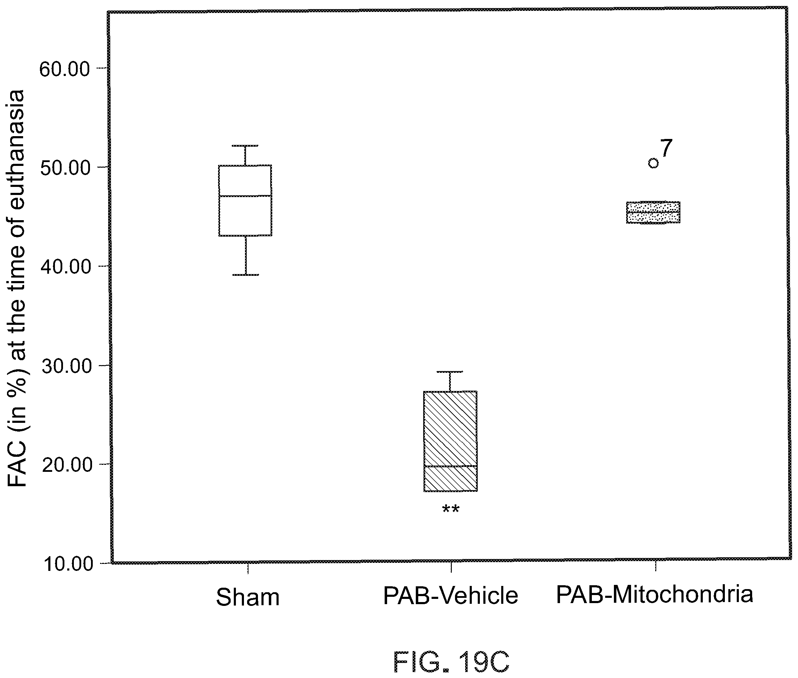

Baseline tricuspid annular plane systolic excursion (TAPSE, in mm) was not different between the groups (C:10.6±0.2, PAB-V:10±0.4, PAB-M: 9.8±0.2; P=0.2), but was significantly lower in the banded groups compared to sham-control 4 weeks after PAB (C:12.3±0.6 versus PAB-V:8.2±0.3 and PAB-M:8±0.3, P<0.001). There was no difference between the two hypertrophy groups prior to treatment with mitochondria (PAB-V versus PAB-M; P=0.9). Four weeks following mitochondrial transplantation, treated hypertrophied hearts were functionally significantly better than vehicle treated hearts (PAB-V:6.7±0.2 versus PAB-M:12.2±0.4; P<0.001) and there was no difference between mitochondria treated hearts and sham-operated controls (C:13±0.5 versus PAB-M: 12.2±0.4; P=0.42). **(****FIGS. 6** **and** **19A****-19C)**

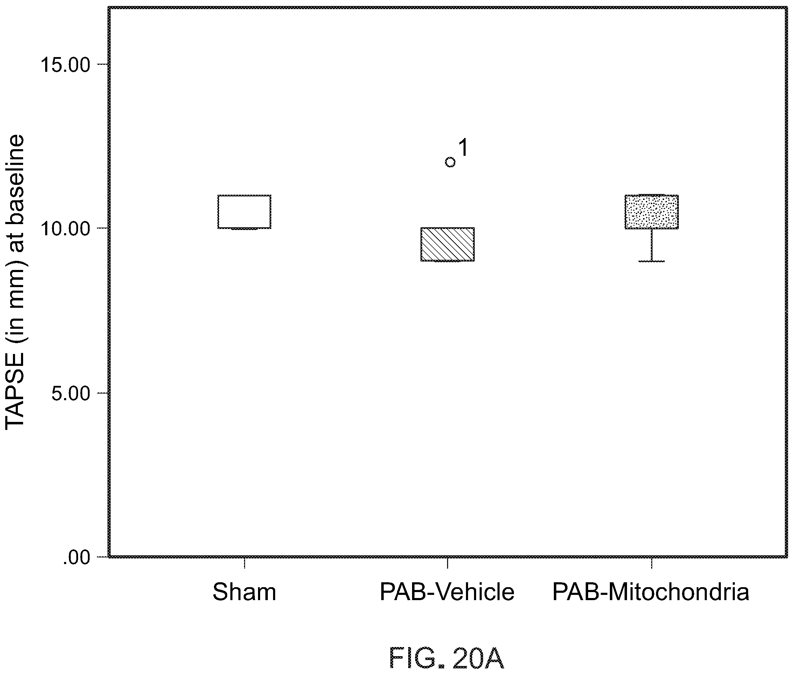

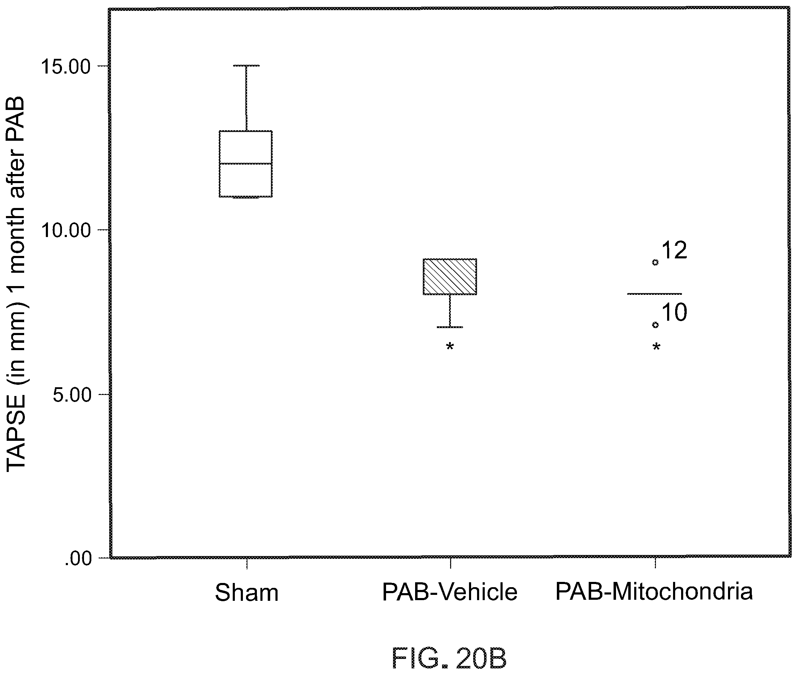

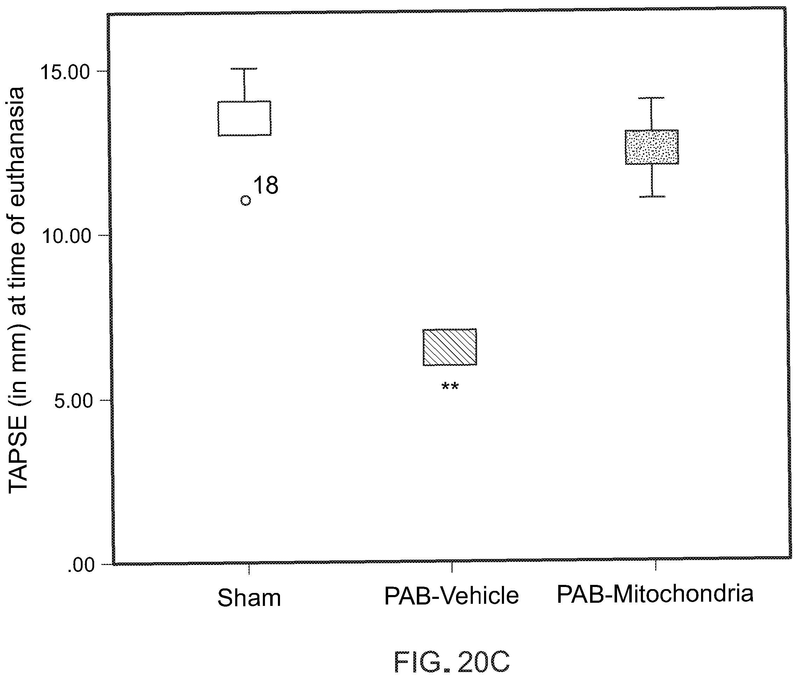

Functional area change (FAC, in %) did not differ between the three groups at baseline (C:38.2±1.4, PAB-S: 41.2±3.4, PAB-M:41.3±2.1; P=0.60), but significantly changed for the hypertrophied groups 4 weeks post-banding compared to sham-operated controls (C:43±1.6, PAB-V: 23.7±1.5, PAB-M: 25±2.5, P<0.001). Both hypertrophy groups were not different from each other prior to mitochondrial treatment but at study endpoint, contractile function of the vehicle treated heart had significantly declined compared to mitochondria treated and sham-operated controls (C: 46.3±1.9 and PAB-M: 45.7±0.9 versus PAB-V: 21.5±1.9; P<0.001. **(****FIGS. 7** **and** **20A****-20C)**

### Invasive Pressure-Volume (PV) Measurements

Vmax (ml/min) and Ved (ml/min) were higher in group PAB-V compared to group C and PAB-M at study endpoint, but differences did not reach significance (C: 83.7±11.8, PAB-V: 122.1±30.3, PAB-M: 94.5±13.8; P=0.42 and C: 73.8±8.3, PAB-V: 99.9±25.3, PAB-M: 84.8+13.6; P=0.57). Pdev (mmHg) was higher in the banded animals at study endpoint compared to sham-operated controls but did not reach significance either (C: 10±0.9, PAB-V: 18.6±6.2, PAB-M: 20.5±1.9, P=0.16).

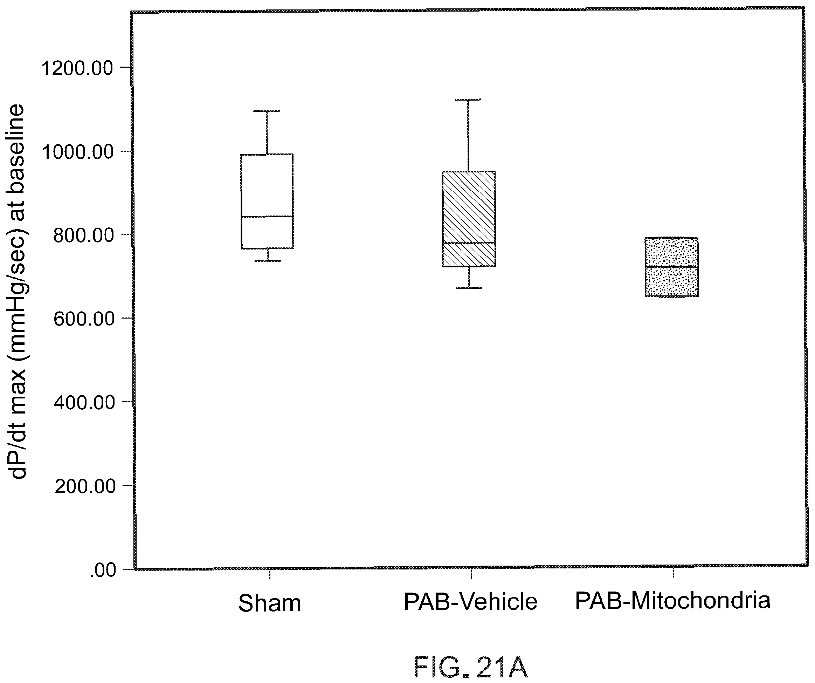

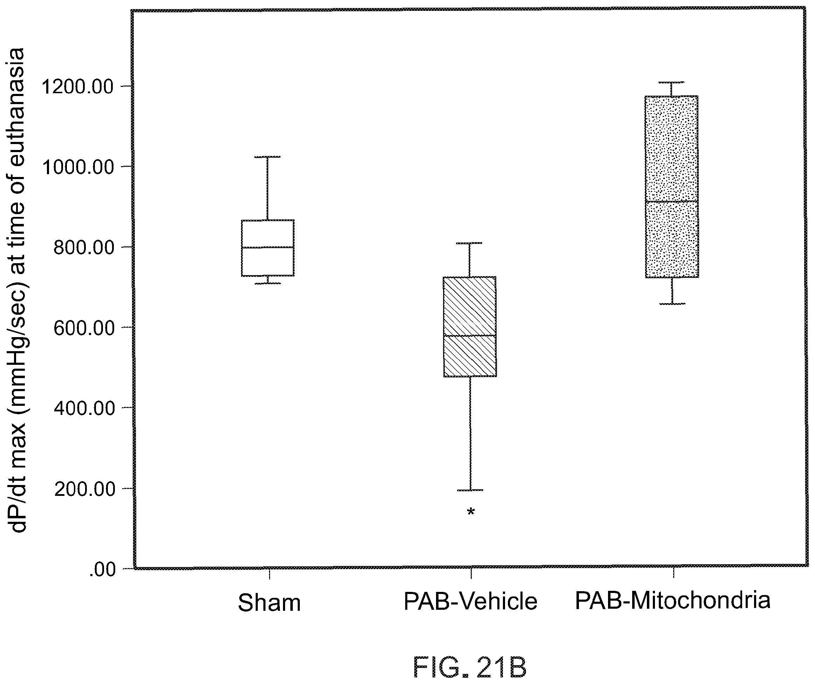

Prior to any intervention all animals started with an average dP/dt max (mmHg/sec) of 831.9±56.8; the animals did not differ significantly from each other at baseline (P>0.05). DP/dt max (mmHg/sec), however, was significantly higher in mitochondria treated hypertrophied hearts compared to vehicle treated hearts (PAB-V: 506.6±88.1 versus PAB-M: 894.9±119.23; P<0.05) at study endpoint, whereas PAB-M hearts did not differ significantly from sham-operated control hearts (C: 777±29.4; P=0.9). **(****FIGS. 9-10** **and** **21A-21B****)**

### Conclusion:

Mitochondrial transplantation maintained hypertrophic adaptation of the RV and preserved contractile function. Addressing the myocardial dysfunction directly by targeting mitochondrial dysfunction can be used to treat patients with pulmonary disease affecting right heart function.

The goal of this study was to target the defect affecting mitochondrial energetics to delay the onset of heart failure. Without being bound by theory, the dysfunction of cardiac mitochondria is pivotal in heart failure in part due to increased energy demands of a thickening RV, our intervention aimed to improve mitochondrial functioning in order to maximize energy production through transplantation of respiration-competent mitochondria. We established that autologous exogenous mitochondria obtained from skeletal muscle sources internalized into hypertrophied cardiomyocytes and increase mitochondrial function equally as well as mitochondria obtained from RV myocardium. Transplantation of autologous mitochondria in a large animal model of pulmonary artery banding, established that cardiomyocytes were preserved from apoptotic cell loss. Furthermore, maintaining hypertrophic growth led to preservation of contractile function compared to untreated hypertrophied hearts which showed signs of dilation and contractile failure. Thus, described and not claimed *per se* are methods of preventing or reducing apoptotic cell loss of cardiomyocytes and preserving or improving contractile function of the heart which comprise administering to a patient a composition comprising mitochondria are described herein.

Without being bound by theory, the right ventricle is the main determinant of prognosis in pulmonary disease associated heart failure. Adaptation and maladaptation of the RV are crucial in the course of the disease. Initially, RV contractility increases through changes in muscle properties and compensatory muscle hypertrophy until a certain point of uncoupling when the afterload exceeds contractility, indicative of maladaptation which is a hallmark of ventricular dilation. In right heart failure, treatment is largely limited to targeting lung function rather than directly interfering in the critical energetic deficit of the RV myocardium which is the result of mitochondrial dysfunction (Hoeper MM, Kramer T, Pan Z, et al. Mortality in pulmonary arterial hypertension: prediction by the 2015 European pulmonary hypertension guidelines risk stratification model. Eur Respir J. 2017;50(2):pii: 1700740; Galie N, Humbert M, Vachiery J-L, et al. 2015 ESC/ERS Guidelines for the diagnosis and treatment of pulmonary hypertension. Eur Resp J. 2015;46:903-975; Tonelli AR, Arelli V, Minai OA, et al. Causes and circumstances of death in pulmonary arterial hypertension. Am J Respir Crit Care Med. 2013;188(3):365-369).

Without being bound by theory, mitochondrial dysfunction resulting in reduced capacity to generate ATP are known to impact heart function since about 90% of cellular ATP is used for contraction and relaxation and calcium regulation (Doenst T, Nguyen TD, Abel ED. Cardiac metabolism in heart failure: Implications beyond ATP production. Circ Res. 2013;113:709-724). Mitochondrial dysfunction can be due to lack of mitochondrial quality control, which leads to defects in metabolic signaling, bioenergetics, calcium transport, reactive oxygen species (ROS) generation, and activation of cell death pathways. This results in a vicious feed-forward cycle that leads to cardiomyocyte cell death from apoptosis (Brown DA, Perry JB, Allen ME, et al. Expert consensus document: mitochondrial function as a therapeutic target in heart failure. Nat Rev Cardiol. 2016;14:238-250). Alterations in mitochondrial function are recognized as the culprit in pressure overload hypertrophy and failure. In human studies of end-stage heart failure, it has been shown that markers for energy metabolism are decreased in the failing heart, which has given rise to the notion that the failing heart is an engine out of fuel (Doenst T, Nguyen TD, Abel ED. Cardiac metabolism in heart failure: Implications beyond ATP production. Circ Res. 2013;113:709-724; Neubauer S. The failing heart-an engine out of fuel. N Engl J Med. 2007;356:1140-1151). However, mitochondria have a more complex role in regulating metabolism and cell death rather than leaving the failing myocardium energy starved. A right heart failure piglet model showed mitochondria had impaired oxidative phosphorylation and significant structural damage underlining the importance of mitochondrial function and structural quality for right heart failure resulting from pressure overload (Noly P-E, Piquereau J, Coblence M, et al. Right ventricular mitochondrial respiratory function in a piglet model of chronic pulmonary hypertension. J Thorac Cardiovasc Surg 2020;159(1):129-140). The data herein indicate that cardiomyocytes were better preserved from apoptotic cell loss following treatment with mitochondria whereas vehicle treated hypertrophied hearts show more apoptotic cell death.

Without being bound by theory, the metabolic demands of a hypertrophied RV are significantly increased and the necessity for the thin RV to increase muscle mass to compensate for increased pressure loading requires enhanced mitochondrial support that needs to occur rapidly. However, mitochondria cannot keep up with rapidly increasing muscle growth (Friehs I, Cowan DB, Choi Y-H, et al. Pressure-overload hypertrophy of the developing heart reveals activation of divergent gene and protein pathways in the left and right ventricular myocardium. Am J Physiol Circ Physiol. 2013;304(5):H697-708; Phillips D, Aponte AM, Covian R, Neufeld E, Yu ZX, Balaban RS. Homogenous protein programming in the mammalian left and right ventricle free walls. Physiol Genomics. 2011;43(21):1198-1206). In our animal model, pressure overload is not reversed and mitochondrial adaptation to a thickening RV muscle is required to maintain function. Based on our results, this compensatory adaptation to accommodate increased pressure loading does not occur long-term as indicated by a decline in wall thickness in vehicle treated hypertrophied hearts. In contrast, mitochondrial transplantation preserves hypertrophic adaptive growth of the RV. Furthermore, the data showed that the source of mitochondria for transplantation is not a determining factor for their benefit. There is no difference which mitochondrial source was used. Skeletal muscle mitochondria responded equally as well as mitochondria isolated from cardiac muscle. Thus, cardiac muscle mitochondria are not necessary for treatment of RVH/RVF-mediated mitochondrial dysfunction. Exogenous autologous skeletal muscle mitochondria preserve contractile function of the failing RV.

Mitochondrial transplantation as a therapeutic intervention targets all aspects of mitochondrial function and structure. Metabolic manipulation of mitochondria alone is not sufficient to treat the failing right ventricle since the structural integrity of the mitochondria must be addressed also. Furthermore, mitochondrial transplantation targets mitochondrial dynamics which are impaired in the hypertrophied/failing right heart. A potential mechanism under review is the disruption of mitochondrial biogenesis, the production of new mitochondria, as an early event in the pathophysiology of heart failure. During early stages of compensated hypertrophy, mitochondrial biogenesis signaling is preserved. In contrast, once decompensated heart failure becomes evident, mitochondrial biogenesis signals decline.

In conclusion, in this study we expanded our understanding of the benefits of mitochondrial transplantation. Particularly, the results show that exogenous autologous skeletal muscle mitochondria preserve contractile function of the failing heart.

### Example 5: Autologous mitochondrial transplantation by intracoronary injection for myocardial protection

Experiments were performed to investigate preischemic intracoronary autologous mitochondrial transplantation (MT) as a therapeutic strategy for prophylactic myocardial protection in a porcine model.

### Methods:

The left coronary artery was cannulated in Yorkshire pigs (n = 26). Mitochondria (1 × 10⁹) or buffer (vehicle [Veh]) were delivered as a single bolus (MTs) or serially (10 injections over 60 minutes; MT_{SS}). Single injections were delivered as a bolus antegrade into the left main coronary artery (1 × 10⁹ in 6 mL). Serial injections (10 injections of 1 × 10⁹ in 6 mL of respiration buffer in each injection) were delivered every 5 minutes. At 15 minutes after injection, the heart was subjected to temporary regional ischemia (RI) by snaring the left anterior descending artery. After 30 minutes of RI, the snare was released, and the heart was reperfused for 120 minutes.

### Results:

Coronary blood flow (CBF) and myocardial function were increased temporarily during the pre-RI period. Following 30 minutes of RI, MT_{S} and MT_{SS} hearts had significantly increased CBF that persisted throughout reperfusion (Veh vs MTs and MTss; P = 0.04). MTs and MTss showed a significantly enhanced ejection fraction (Veh vs MTs, P < 0.001; Veh vs MTss, P = 0.04) and developed pressure (Veh vs MT_{S}, P < 0.001; Veh vs MT_{SS}, P =0.03). Regional function, assessed through segmental shortening (Veh vs MT_{S}, P = 0.03; Veh vs MT_{SS}, P < 0.001), fractional shortening (Veh vs MT_{S}, P < 0.001; Veh vs MT_{SS}, P = 0.04), and strain analysis (Veh vs MT_{S}, P = 0.002; Veh vs MT_{SS}, P = 0.003), was also significantly improved. Although there was no difference in the area at risk between treatment groups, infarct size was significantly reduced in both MT groups (Veh vs MTs and MTss, P < 0.001).

### Conclusions:

Pre-ischemic MT by single or serial intracoronary injections provides prophylactic myocardial protection, significantly decreasing infarct size and enhancing global and regional heart function.

### Example 6: Myocardial Protection by Intracoronary Delivery of Mitochondria

Autologous mitochondrial transplantation involves supplying the ischemic tissue with viable, respiration-competent mitochondria isolated from one's own body to mitigate the effects of native mitochondrial damage. Experiments were performed to investigate the safety and efficacy of intracoronary delivery of mitochondria in the clinically relevant swine model.

### Methods:

Adult swine were anesthetized, and autologous mitochondria were isolated. Animals were sedated with Telazol (2.2-4.4 mg/kg)/Xylazine (1-2 mg/kg) and intubated. General anesthesia was maintained with 0.5-2% isoflurane-oxygen mixture. Median sternotomy was performed, and the heart was suspended in a pericardial cradle. Then, angiographic access to the left coronary artery (LCA) was established by floating a 5F JR angiography catheter (Merit Medical Sys, UT) through the right carotid artery (5F sheath) to the left coronary ostium under fluoroscopy. Uptake and biodistribution of mitochondria were evaluated by angiographic injection of left coronary artery with ¹⁸F-rhodamine-6G-labeled mitochondria followed by position emission tomography (PET) (n=3). Safety profile of intracoronary mitochondrial injection was evaluated under normal condition, during coronary vasoconstriction and during tachycardia (n=18). To assess therapeutic efficacy of intracoronary mitochondrial transplantation, left anterior descending artery was snared for 30 minutes. At the onset of reperfusion, animals received either mitochondria (n=8) or vehicle solution (n=8) followed by 2 hours of reperfusion.

### Results:

Intracoronary delivery of mitochondria resulted in rapid uptake and specific biodistribution of mitochondria throughout the heart. Coronary patency and myocardial function were preserved under all tested conditions. Intracoronary injection of mitochondria resulted in a concentration-dependent increase in coronary blood flow (CBF). Mitochondria-induced hyperemia required mitochondrial viability, ATP production and in part, activation of vascular inwardly-rectifying potassium channels (K_{IR}). Intracoronary mitochondrial delivery resulted in significant enhancement of post-ischemic myocardial function, improvement of CBF and reduction of infarct size compared to controls.

Intracoronary mitochondrial transplantation is a safe and efficacious method for improving myocardial perfusion, myocardial function and heart tissue survival.

### OTHER EMBODIMENTS

It is to be understood that while the invention has been described in conjunction with the detailed description thereof, the foregoing description is intended to illustrate and not limit the scope of the invention, which is defined by the scope of the appended claims.

## Claims

1. A composition comprising isolated mitochondria or a combined mitochondrial agent that comprises isolated mitochondria for use in the treatment or prevention of heart failure in a patient having pulmonary disease, wherein said composition is to be administered in a therapeutically effective amount to the patient, and wherein the patient has or is at risk of developing heart failure-right ventricular hypertrophy (RVH) or right ventricular failure (RVF).

2. The composition for use of claim 1, wherein the pulmonary disease affects right ventricular function.

3. The composition for use of claim 1, wherein the composition is to be injected into a blood vessel of the patient.

4. The composition for use of claim 1, wherein the mitochondria are autogeneic, allogeneic or xenogeneic.

5. A composition comprising isolated mitochondria or a combined mitochondrial agent that comprises isolated mitochondria for use in maintaining right ventricular (RV) contractility, maintaining RV capillary density, preventing RV dilatation, or delaying the onset of RVF in a patient, wherein said composition is to be administered in a therapeutically effective amount to the patient, wherein the patient has a pulmonary disease and has or is at risk of developing heart failure-right ventricular hypertrophy (RVH) or right ventricular failure (RVF), and wherein said pulmonary disease affects right ventricular function.

6. A composition comprising isolated mitochondria or a combined mitochondrial agent that comprises isolated mitochondria for use in the treatment of heart hypertrophy, delaying of the onset of heart hypertrophy, reduction of the risk of developing heart hypertrophy in a patient, wherein said composition is to be administered in a therapeutically effective amount to the patient, wherein the patient has a pulmonary disease and wherein the patient has or is at risk of developing RVH or RVF.

7. The composition for use of claim 1, 5, or 6, wherein the composition is to be administered to the patient by intramyocardial injection.

8. The composition for use of any one of claims 1-6, wherein the patient has pulmonary hypertension, and wherein the patient has or is at risk of developing RVH.

## Patentansprüche

1. Zusammensetzung, umfassend isolierte Mitochondrien oder einen mitochondrialen Kombinationswirkstoff, der isolierte Mitochondrien umfasst, zur Verwendung bei der Behandlung oder Vorbeugung von Herzinsuffizienz bei einem Patienten mit Lungenkrankheit, wobei die Zusammensetzung in einer therapeutisch wirksamen Menge dem Patienten zu verabreichen ist und wobei bei dem Patienten Herzinsuffizienz-rechtsventrikuläre Hypertrophie (RVH) oder Rechtsherzinsuffizienz (RVF) vorliegt oder ein Risiko der Entwicklung davon besteht.

2. Zusammensetzung zur Verwendung nach Anspruch 1, wobei die Lungenkrankheit eine Auswirkung auf die Funktion der rechten Herzkammer hat.

3. Zusammensetzung zur Verwendung nach Anspruch 1, wobei die Zusammensetzung in ein Blutgefäß des Patienten zu injizieren ist.

4. Zusammensetzung zur Verwendung nach Anspruch 1, wobei die Mitochondrien autogen, allogen oder xenogen sind.

5. Zusammensetzung, umfassend isolierte Mitochondrien oder einen mitochondrialen Kombinationswirkstoff, der isolierte Mitochondrien umfasst, zur Verwendung beim Aufrechterhalten von rechtsventrikulärer (RV-) Kontraktilität, Aufrechterhalten von RV-Kapillardichte, Verhindern von RV-Dilatation oder Verzögern des Beginns von RVF bei einem Patienten, wobei die Zusammensetzung in einer therapeutisch wirksamen Menge dem Patienten zu verabreichen ist, wobei der Patient an einer Lungenkrankheit leidet und bei ihm Herzinsuffizienz-rechtsventrikuläre Hypertrophie (RVH) oder Rechtsherzinsuffizienz (RVF) vorliegt oder ein Risiko der Entwicklung davon besteht und wobei die Lungenkrankheit eine Auswirkung auf die Funktion der rechten Herzkammer hat.

6. Zusammensetzung, umfassend isolierte Mitochondrien oder einen mitochondrialen Kombinationswirkstoff, der isolierte Mitochondrien umfasst, zur Verwendung bei der Behandlung von Herzhypertrophie, Verzögerung des Beginns von Herzhypertrophie, Verringerung des Risikos, Herzhypertrophie zu entwickeln, bei einem Patienten, wobei die Zusammensetzung in einer therapeutisch wirksamen Menge dem Patienten zu verabreichen ist, wobei der Patient an einer Lungenkrankheit leidet und wobei bei dem Patienten RVH oder RVF vorliegt oder ein Risiko der Entwicklung davon besteht.

7. Zusammensetzung zur Verwendung nach Anspruch 1, 5 oder 6, wobei die Zusammensetzung dem Patienten durch intramyokardiale Injektion zu verabreichen ist.

8. Zusammensetzung zur Verwendung nach einem der Ansprüche 1-6, wobei der Patient an pulmonaler Hypertonie leidet und wobei bei dem Patienten RVH vorliegt oder ein Risiko der Entwicklung davon besteht.

## Revendications

1. Composition comprenant des mitochondries isolées ou un agent mitochondrial combiné qui comprend des mitochondries isolées pour utilisation dans le traitement ou la prévention de l'insuffisance cardiaque chez un patient atteint de maladie pulmonaire, dans laquelle ladite composition doit être administrée en une quantité thérapeutiquement efficace au patient, et dans laquelle le patient a ou risque de développer une hypertrophie ventriculaire droite (RVH) ou une insuffisance ventriculaire droite (RVF).

2. Composition pour utilisation selon la revendication 1, dans laquelle la maladie pulmonaire affecte la fonction ventriculaire droite.

3. Composition pour utilisation selon la revendication 1, dans laquelle la composition doit être injectée dans un vaisseau sanguin du patient.

4. Composition pour une utilisation selon la revendication 1, dans laquelle les mitochondries sont autogéniques, allogéniques ou xénogéniques.

5. Composition comprenant des mitochondries isolées ou un agent mitochondrial combiné qui comprend des mitochondries isolées pour une utilisation dans le maintien de la contractilité ventriculaire droite (RV), le maintien de la densité capillaire RV, la prévention de la dilatation RV ou le retardement de l'apparition de la RVF chez un patient, ladite composition devant être administrée en une quantité thérapeutiquement efficace au patient, le patient présentant une maladie pulmonaire et présentant ou risquant de développer une insuffisance cardiaque - hypertrophie ventriculaire droite (RVH) ou une insuffisance ventriculaire droite (RVF), et où ladite maladie pulmonaire affecte la fonction ventriculaire droite.

6. Composition comprenant des mitochondries isolées ou un agent mitochondrial combiné qui comprend des mitochondries isolées destinées à être utilisées dans le traitement de l'hypertrophie cardiaque, le retardement de l'apparition de l'hypertrophie cardiaque, la réduction du risque de développer une hypertrophie cardiaque chez un patient, dans laquelle ladite composition doit être administrée en une quantité thérapeutiquement efficace au patient, dans laquelle le patient a une maladie pulmonaire et dans laquelle le patient a ou est à risque de développer une RVH ou une RVF.

7. Composition pour utilisation selon la revendication 1, 5 ou 6, dans laquelle la composition doit être administrée au patient par injection intramyocardique.

8. Composition pour utilisation selon l'une quelconque des revendications 1 à 6, dans laquelle le patient a une hypertension pulmonaire, et dans laquelle le patient a ou est à risque de développer une RVH.
